# EUROPEAN PATENT SPECIFICATION

(11) **EP 1 955 079 B1**
(45) Date of publication and mention of the grant of the patent: **18.08.2010**
(21) Application number: 06836307.6
(22) Date of filing: 16.10.2006
(51) Int. Cl.: G01N 35/00, C12Q 1/68

(54) **COMPACT APPARATUS, COMPOSITIONS AND METHODS FOR PURIFYING NUCLEIC ACIDS**
KOMPAKTE VORRICHTUNG, ZUSAMMENSETZUNGEN UND VERFAHREN ZUR REINIGUNG VON NUKLEINSÄUREN
APPAREIL COMPACT, COMPOSITIONS ET PROCEDES POUR LA PURIFICATION D'ACIDES NUCLEIQUES

(30) Priority: 21.10.2005 US 255807
(43) Date of publication of application: 13.08.2008
(73) Proprietor: QIAGEN North American Holdings, Inc., Germantown, MD 20874 (US)
(72) Inventor: QUINN, Tim, St. Michael, MN 55376 (US); WIRBISKY, Alan, Eden Prairie, MN 55344 (US); VAN DER LUGHT, Nick, Eagan, MN 55123 (US); MORKEN, Nate, Maple Grove, MN 55311 (US); JOHNSON, Rebecca, St. Louis Park, MN 55426 (US); PAULSEN, Kim, Brooklyn Park, MN 55443 (US); STROM, Dan, Minneapolis, MN 55417 (US); BALDRICA, Jim, Minneapolis, MN 55414 (US)
(74) Representative: Polypatent
(86) International application number: PCT/US2006/040173
(87) International publication number: WO 2007/050327

(56) References cited:
- EP-A- 1 441 225
- EP-A2- 0 818 461
- WO-A-00/34463
- WO-A-93/25913
- WO-A-03/033739
- WO-A-2004/090132
- WO-A-2004/094635
- WO-A-2005/007895
- WO-A-2005/026347
- WO-A-2005/092911
- WO-A-2006/052680
- DE-A1- 10 063 984
- US-A1- 5 443 791
- US-A1- 6 060 022
- US-A1- 2003 073 830
- US-A1- 2003 180 754
- US-A1- 2005 032 105
- US-B1- 6 605 213

## Description

This invention relates to materials and methods for isolating RNA or DNA from a biological sample.

Nucleic acids such as deoxyribonucleic acid (DNA) and ribonucleic acid (RNA) are used extensively in the field of molecular biology for research and clinical analyses. A number of methods exist for isolating DNA and RNA that entail disruption of cells and liberating nucleic acids into a solution. RNA is highly sensitive to degradation. Therefore, methods also exist for protecting RNA from enzymatic digestion by RNA degrading enzymes (*e.g*., RNases). The RNA can then be separated from the DNA and protein. The isolation processes for DNA and RNA are usually performed in a stepwise fashion, wherein cells are lysed under conditions that inhibit DNases or RNases, and the nucleic acid purified from contaminating cellular material either by binding to a solid phase with subsequent washing to remove contaminants or by selective precipitation to partition the nucleic acids away from the contaminants.

Additionally, instruments are available that automate the steps of isolating and purifying nucleic acid. Methods used by such instruments can include use of solid supports, such as glass fiber columns and other membranes, or use of magnetic particles in combination with chaotropic salts such as guanidine, or they can use liquid-phase methods such as phenol/chloroform or salting-out methods.

EP 0 818 461 A2 describes a method for isolating a ribonucleic acid, which comprises dissolving of a sample containing the ribonucleic acid in an acidic solution which contains a lithium salt and a chaotropic agent, then bringing the ribonucleic acid into contact with a nucleic acid-binding carrier to allow selective adsorption of the ribonucleic acid onto said carrier, afterwards separating the ribonucleic acid-bound carrier from the liquid phase and finally eluting the ribonucleic acid from the carrier.

US 2003/0180754 A1 describes a method of isolating nucleic acid from a blood sample, said method comprising selectively isolating leucocytes from said sample by binding the leucocytes to a solid support through the action of a leucocyte-specific binding partner attached to the solid support, lysing the isolated leucocytes and finally binding nucleic acids released from said lysed leucocytes to said solid support.

WO 2004/090132 A2 discloses an automated method for separating genomic nucleic acid of a cell from nucleic acid having a lower molecular weight than the genomic nucleic acid directly from a cell growth culture, as well as a lysate solutions to be used in such a method.

WO 03/033739 A1 discloses compositions and methods for purifying RNA using a solid support.

US 2005/0032105 A1 and WO 2004/094635 A2 disclose compositions and methods for manually purifying DNA and RNA, respectively, using a solid support.

WO 2005/026347 A1 and WO 2005/007895 A1 both disclose manual methods for lysing cells and isolating nucleic acid from the lysate using a solid support as well as a lysis solution comprising a high amount of chaotropic salts.

The formulations and methods featured by the present invention allow for the economical extraction and purification of RNA, DNA, or both RNA and DNA from the same sample, thus providing an advantage to users.

The present invention provides an automated, method for isolating DNA from a biological sample containing DNA, which has three steps. The first step involves mixing a first reagent (a Lysis reagent) having a pH of at least 9, a sample, and magnetic particles (which may be in glycerol, with or without Proteinase K also present) to lyse the cells in the sample and thus release nucleic acid from the sample. The lysing step may take place in the presence of heat The first (Lysis) reagent contains (1) a lithium salt at a concentration of about 2-5 M; (2) a surfactant at a concentration of about 20-40% by volume; (3) a buffer; (4) a chelating agent at about 5-20 mM; (5) a detergent at about 0.05 - 2%; (6) an antifoaming agent at about 0.005-0.1% by volume, to form a lysate. The second step involves adding a second reagent to the lysate, such that the DNA quantitatively binds to the magnetic particles, wherein the second reagent contains (1) a lithium salt at a concentration of at least 9 M; (2) a surfactant concentration of at least 5%, and (3) a buffer. The third step involves applying a magnetic force to the magnetic processing tube, wherein the DNA is liberated from the sample.

The present invention provide an automated method for isolating DNA or RNA from a biological Lysate containing DNA or RNA, which has the three steps. First, one mixes a first reagent (a Lysis solution) having a pH of at least 7 into a sample and homogenizing the sample so that the cells in the sample are lysed to release nucleic acid from the sample to form a lysate, and mixing the Lysate with magnetic particles in glycerol in a magnetic processing tube. In this embodiment, the Lysis reagent contains (1) a lithium salt at a concentration of about 2-10 M; (2) a surfactant at a concentration of about 5-20% by volume; (3) a buffer; (4) a chelating agent at about 5-20 mM; (5) a detergent at about 0.05 - 5%; and (6) an antifoaming agent at about 0.005-0.1% by volume. Second, one optionally adds a second reagent to the lysate, such that DNA or RNA quantitatively bind to the magnetic particles; wherein the second reagent contains (1) a lithium salt at a concentrations of at least 5 M; (2) a surfactant concentration of at least 5%; (3) a buffer, and (4) optionally, an alcohol such as ethanol or isopropanol, or the second reagent may be an alcohol such as ethanol or isopropanol. Third, one applies a magnetic force to the magnetic processing tube, wherein the DNA or RNA is liberated from the sample. In certain embodiments, the buffered binding solution contains a lithium salt at a concentration of at least 9 M, a surfactant at a concentration of at least 10%, and a pH of 8. In certain embodiments, the alcohol binding solution comprises lithium salt at a concentration of at least 5 M and an alcohol at a concentration of at least 55% v/v.

The present invention provides lysis solutions for lysing cells containing a lithium salt (*e.g*., lithium chloride or lithium bromide) at a concentration of about 2 M to about 10 M, a surfactant of about 5 % to about 40%, a buffer (*e.g*., Tris), wherein the solutions have pH values of at least 7 to about 11, each containing a chelating agent of about 5 mM to 20 mM, each containing a detergent of about 0.05 % to about 2 %, and each containing an antifoam agent (e.g., Dow Corning^{®} Antifoam A compound) of about 0.005 % to about 0.1 %. In certain embodiments, the lysis solution may contain 20 mM TCEP. In certain embodiments, the surfactant is diethylene glycol monoethyl ether (DGME). In certain embodiments the buffer is Tris buffer. In certain embodiments, the solution has a pH of at least about 9, or even at least about 11. In certain embodiments the detergent is a non-ionic detergent, such as a Tween class detergent, a Triton class detergent, a Tergitol detergent, Nonidets or Igepal. In certain embodiments, the detergent is an anionic detergent, such as SDS (sodium dodecyl sulfate). In certain embodiments the surfactant is diethylene glycol monoethyl ether (DGME). In certain embodiments, the lithium salt is present at a concentration of about 2 M to about 5 M. In certain embodiments, the lysis solution contains 50 mM Tris (pH 10-11), 2 M LiCl, 0.1% SDS, 30% DGME,10 mM citrate and 0.05% Dow Corning^{®} Antifoam A compound. In an alternative embodiment, the lysis solution contains 100 mM Tris (pH 7), 8 M LiCl, 0.1% Triton X, 10% DGME, 10 mM EDTA and 0.01% Dow Corning®Antifoam A compound.

In certain embodiments the buffer is citrate buffer. In certain embodiments, the chelating agent is ethylenediaminetetraacetic acid (EDTA). In certain embodiments, the chelating agent is citrate. In certain embodiments, the detergent is a nonionic detergent, such as a Tween class detergent, a Triton class detergent, a Tergitol detergent, Nonidets or Igepal. In certain embodiments the detergent is an ionic detergent, such as the anionic detergent sodium dodecyl sulfate (SDS). In certain embodiments the antifoam agent is Dow Corning^{®} Antifoam A compound.

A process assembly that can be used in the methods of the present invention contains a magnetic processing assembly; and a magnetic elevator assembly, wherein the magnetic processing assembly is configured to hold at least one tube (*e.g*., a conical tube), wherein the magnetic elevator assembly has a magnet mounting assembly, wherein the magnet mounting assembly has at least one magnet that is able to assume at least a first position and a second position, wherein in the first position, the at least one magnet is moved distally from the magnetic processing assembly and wherein in the second position, the at least one magnet is moved adjacent to the tube when the tube is in the magnetic processing tube assembly. The magnet mounting assembly may be able to assume the at least first and second positions using a drive system. In certain embodiments of this magnetic processing assembly, the drive system may have either or both a pivoting bar and/or one or more springs.

A pump assembly that can be used in the method of the present invention contains at least one cylinder having a piston, a cylinder actuator rod, and an upper and lower port for fluidly connecting the cylinder to one or more pipettor assemblies.

In the method of the present invention an apparatus for nucleic acid isolation can be used that contains (a) a process assembly that has a mounting plate, wherein the mounting plate has (i) at least one holder for one or more tubes; (ii) a receiving mechanism for receiving the magnetic processing assembly; and (iii) a first and second receptacle for receiving reagents; (b) an upper deck assembly containing the pump assembly described above that contains a Y-direction drive mechanism; (c) an aspiration pipettor assembly; and (d) a dispense pipettor assembly, wherein the aspiration pipettor assembly and the dispense pipettor assembly are fluidly connected to the Y-direction drive mechanism. The mounting plate further may have a holder for at least one pipette tip. The mounting plate may have at least two holders for tubes (*e.g*., one holder for output tubes and the other holder for waste tubes). The reagents may be contained within one or more reagent packs. The apparatus also has a magnetic processing assembly.

A system for automated isolation of nucleic acids that can be used in the methods of the present invention has as one element the apparatus described above, and an electronic control system, wherein the electronic control system contains a microcontroller (*e.g*., a programmable microcontroller) and a user interface (*e.g*., a display). The apparatus also has a magnetic processing assembly.

The automated methods for isolating nucleic acid from a biological sample containing nucleic acid may comprise the following steps: (a) adding a sample containing a biological starting material into a magnetic processing tube, wherein the magnetic processing tube contains silica coated magnetic particles, and optionally an enzyme solution; and (b) activating an apparatus for isolating nucleic acids, wherein the apparatus comprises a dispense pipettor assembly; a aspiration pipettor assembly; reagent pack comprising the lysis solution, an optional nucleic acid binding solution, a wash I solution, an optional wash II solution, a wash III solution, an optional wash IV solution, and elution solution; disposable tips; an output tube, a waste tube; magnetic elevator assembly; and a magnetic processing tube, wherein the apparatus carries out the following steps:
(1) optionally verifying the presence and/or volume of the sample by a sensing means;
(2) transferring the lysis solution from the reagent pack to the magnetic processing tube by means of the dispense pipettor assembly and incubating the samples with heat;
(3) optionally transferring nucleic acid binding solution from the reagent pack to the magnetic processing tube by means of the dispense pipettor assembly, contacting the magnetic processing tube with the magnetic elevator assembly, and aspirating liquid from the magnetic processing tube to the waste tube;
(4) transferring wash I solution from the reagent pack to the magnetic processing tube by means of the dispense pipettor assembly, contacting the magnetic processing tube with the magnetic elevator assembly, and aspirating liquid from the magnetic processing tube to the waste tube;
(5) optionally transferring wash II solution from the reagent pack to the magnetic processing tube by means of the dispense pipettor assembly, contacting the magnetic processing tube with the magnetic elevator assembly, and aspirating liquid from the magnetic processing tube to the waste tube;
(6) transferring wash III solution from the reagent pack, one or more times, to the magnetic processing tube by means of the dispense pipettor assembly, contacting the magnetic processing tube with the magnetic elevator assembly, and aspirating liquid from the magnetic processing tube to the waste tube;
(7) transferring elution solution from the reagent pack to the magnetic processing tube by means of the dispense pipettor assembly to form a purified nucleic acid sample, contacting the magnetic processing tube with the magnetic elevator assembly; and
(8) transferring the purified nucleic acid sample from the magnetic processing tube by means of the aspiration pipettor assembly to an output tube.

The sample is mixed by the aspiration pipettor assembly after the dispensing pipettor assembly dispenses a solution. The aspiration pipettor assembly accomplishes this mixing by pipetting the sample up and down.

In certain embodiments, the sample to be isolated is DNA, RNA or both DNA and RNA.

In certain embodiments, the binding solution contains 50 to 150 mM Tris at a pH of about 7 to 10, a complexing salt at a concentration of about 5 to 15 M, and a surfactant at a concentration of about 5 to 15%. The complexing salt may be lithium chloride. The solution may further contain an alcohol. In an alternative embodiment the binding solution only contains an alcohol.

The enzyme solution may contain about 10 to 25 mg/mL Proteinase K and/or about 2 to 20 mg/mL RNase A.

The method may involve the following steps after step (6):
(i) transferring DNase I solution and Rebinding solution from the reagent pack to the magnetic processing tube by means of the dispense pipettor assembly, contacting the magnetic processing tube with the magnetic elevator assembly, and aspirating liquid from the magnetic processing tube to the waste tube;
(ii) transferring wash IV solution from the reagent pack, one or more times, to the magnetic processing tube by means of the dispense pipettor assembly and mixing, contacting the magnetic processing tube with the magnetic elevator assembly, aspirating liquid from the magnetic processing tube to the waste tube;
(iii) transferring wash III solution from the reagent pack, one or more times, to the magnetic processing tube by means of the dispense pipettor assembly and mixing, contacting the magnetic processing tube with the magnetic elevator assembly, and aspirating liquid from the magnetic processing tube to the waste tube.

The DNase I solution may contain DNase I present at a concentration of about 0.25 to 1.0 U/µL.

The rebinding solution may comprise salt, such as lithium chloride, at a concentration of about 5 to 15 M (*e.g*., 5, 6, 7, 8, 9, 10, 11, 12, 13 , 14, or 15 M) and/or an alcohol such as ethanol or isopropanol, at about 20 to 100 % v/v (*e.g*., 20, 30, 40, 50, 60, 70, 80, 90, 100% v/v).

The lysis solution may contain a lithium salt at a concentration of about 2 M to about 9 M, one or more amphiphilic reagents at a concentration of at least 10-40% w/v, and a buffer, wherein the solution has a pH of at least 7.

The wash I solution may contain lithium salt at a concentration between 4-10 M (*e.g*., 5 M LiCI), and an alcohol at a concentration of about 15-80 % v/v (*e.g*., 55% methanol).

The wash II solution may represent an alcohol (*e.g*., 100% isopropyl alcohol at a concentration of 100% v/v).

The wash III solution may contain a buffer (*e.g*., 50 to 150 mM Tris at a pH of about 6 to 9), 50 to 90% v/v starting concentration of alcohol, and a chelator (*e.g*., 1 to 20 mM EDTA). In one example, the wash III solution comprises 70% ethanol (starting concentration, v/v), 100 mM Tris (pH 6-8) and 5mM EDTA.

The wash IV solution may comprise 4-10 M lithium salt (*e.g*., 6 M LiCl), an alcohol at a concentration of about 5 to 30 % v/v (*e.g*., 18% methanol, final concentration).

The magnetic particles may be packaged in a mixture containing glycerol, and the mixture may further contain a buffer.

The magnetic elevator assembly may have a magnet mounting assembly and at least one magnet operably linked to the magnet mounting assembly, wherein the at least one magnet that is able to assume at least a first position and a second position, wherein in the first position, the at least one magnet is moved distally from a magnetic processing tube holder and wherein in the second position, the at least one magnet is moved adjacent to the magnetic processing tube holder.

The precision pump may have at least one cylinder comprising a piston; a cylinder actuator rod; and an upper and lower port for fluidly connecting the cylinder to one or more pipettor assemblies.

The DNA Direct Lysis protocol that may be used in the method of the present invention has the following steps: (a) adding a sample containing a biological starting material into a magnetic processing tube; and (b) activating an apparatus for isolating nucleic acids, using a Direct Lysis Protocol for DNA, where the apparatus has a dispense pipettor assembly; a aspiration pipettor assembly; reagent pack containing Lysis solution, Binding solution, Wash I solution, Wash II solution, Wash III solution and Elution solution; disposable tips; an output tube, a waste tube; magnetic elevator assembly; and a magnetic processing tube containing magnetic particles such as silica coated magnetic particles, buffer, glycerol and Proteinase K. The apparatus carries out the following steps:
(1) verifying the presence and volume of the sample by a sensing means;
(2) transferring lysis solution from the reagent pack to the magnetic processing tube by means of the dispense pipettor assembly, and incubating the samples with heat;
(3) transferring DNA binding solution from the reagent pack to the magnetic processing tube by means of the dispense pipettor assembly and mixing, contacting the magnetic processing tube with the magnetic elevator assembly, and aspirating liquid from the magnetic processing tube to the waste tube;
(4) transferring wash I solution one or more times from the reagent pack to the magnetic processing tube by means of the dispense pipettor assembly and mixing, contacting the magnetic processing tube with the magnetic elevator assembly, and aspirating liquid from the magnetic processing tube to the waste tube;
(5) transferring wash II solution from the reagent pack to the magnetic processing tube by means of the dispense pipettor assembly and mixing, contacting the magnetic processing tube with the magnetic elevator assembly, and aspirating liquid from the magnetic processing tube to the waste tube;
(6) transferring wash III solution from the reagent pack, one or more times, to the magnetic processing tube by means of the dispense pipettor assembly and mixing, contacting the magnetic processing tube with the magnetic elevator assembly, aspirating liquid from the magnetic processing tube to the waste tube;
(7) transferring elution solution from the reagent pack to the magnetic processing tube by means of the dispense pipettor assembly and mixing, to elute a purified nucleic acid sample, contacting the magnetic processing tube with the magnetic elevator assembly; and
(8) transferring the purified DNA sample from the magnetic Processing tube by means of the aspiration pipettor assembly to an output tube.

The DNA Lysate protocol that may be used in the method of the present invention has the following steps: (a) adding the lysate containing DNA into a magnetic processing tube, and (b) activating an apparatus for isolating nucleic acids, using a Lysate Protocol for DNA, where the apparatus has a dispense pipettor assembly; a aspiration pipettor assembly; a bottled Lysis solution, RNase reagent, a reagent pack containing an optional Binding solution, Wash I solution, Wash III solution, and Elution solution; disposable tips; an output tube, a waste tube; magnetic elevator assembly; and a magnetic processing tube containing silica coated magnetic particles, buffer, and glycerol. The apparatus carries out the following steps:
(1) verifying the presence and volume of the sample by a sensing means;
(2) adding an optional binding solution, contacting the magnetic processing tube with the magnetic elevator assembly, and aspirating liquid from the magnetic processing tube to the waste tube;
(3) transferring wash I solution one or more times, from the reagent pack to the magnetic processing tube by means of the dispense pipettor assembly and mixing, contacting the magnetic processing tube with the magnetic elevator assembly, aspirating liquid from the magnetic processing tube to the waste tube;
(4) transferring wash III solution from the reagent pack, one or more times, to the magnetic processing tube by means of the dispense pipettor assembly and mixing, contacting the magnetic processing tube with the magnetic elevator assembly, aspirating liquid from the magnetic processing tube to the waste tube;
(5) transferring elution solution from the reagent pack to the magnetic processing tube by means of the dispense pipettor assembly and mixing, to elute a purified nucleic acid sample, contacting the magnetic processing tube with the magnetic elevator assembly; and
(6) transferring the purified DNA sample from the magnetic processing tube by means of the aspiration pipettor assembly to an output tube.

A RNA Lysate protocol that may be used in the method of the present invention has the following steps: (a) adding the lysate containing the nucleic acids (total nucleic acids) into a magnetic processing tube, and (b) activating an apparatus for isolating nucleic acids, using a Lysate Protocol for RNA, where the apparatus has a dispense pipettor assembly; a aspiration pipettor assembly; a bottled Lysis solution, a reducing agent, a reagent pack containing a Binding solution, Wash I solution, Wash III solution, DNase solution, Rebinding solution, Wash IV solution, and elution solution; disposable tips; an output tube, a waste tube; magnetic elevator assembly; and a magnetic processing tube containing silica coated magnetic particles, buffer, and glycerol. The apparatus carries out the following steps:
(1) verifying the presence and volume of the sample by a sensing means;
(2) adding a binding solution, contacting the magnetic processing tube with the magnetic elevator assembly, and aspirating liquid from the magnetic processing tube to the waste tube;
(3) transferring wash I solution from the reagent pack to the magnetic processing tube by means of the dispense pipettor assembly and mixing, contacting the magnetic processing tube with the magnetic elevator assembly and aspirating liquid from the magnetic processing tube to the waste tube;
(4) transferring wash III solution from the reagent pack, to the magnetic processing tube by means of the dispense pipettor assembly and mixing, contacting the magnetic processing tube with the magnetic elevator assembly, and aspirating liquid from the magnetic processing tube to the waste tube;
(5) transferring DNase solution from the reagent pack, to the magnetic processing tube by means of the dispense pipettor assembly and mixing;
(6) transferring Rebinding solution from the reagent pack, to the magnetic processing tube by means of the dispense pipettor assembly and mixing, contacting the magnetic processing tube with the magnetic elevator assembly, and aspirating liquid from the magnetic processing tube to the waste tube;
(7) transferring wash IV solution from the reagent pack, one or more times, to the magnetic processing tube by means of the dispense pipettor assembly and mixing, contacting the magnetic processing tube with the magnetic elevator assembly, and aspirating liquid from the magnetic processing tube to the waste tube;
(8) transferring wash III solution from the reagent pack, one or more times, to the magnetic processing tube by means of the dispense pipettor assembly and mixing, contacting the magnetic processing tube with the magnetic elevator assembly, and aspirating liquid from the magnetic processing tube to the waste tube;
(9) transferring elution solution from the reagent pack to the magnetic processing tube by means of the dispense pipettor and mixing, to elute a purified nucleic acid sample, and contacting the magnetic processing tube with the magnetic elevator assembly; and (10) transferring the purified RNA sample from the magnetic processing tube by means of the aspiration pipettor assembly to an output tube.

The magnetic particles may be packaged in a mixture containing glycerol, and may further contain a buffer and enzymes, which are dried in order to immobilize the beads at the bottom of the tube and stabilize the enzymes. The magnetic elevator assembly may have a magnet mounting assembly and at least one magnet operably linked to the magnet mounting assembly, wherein the at least one magnet that is able to assume at least a first position and a second position, wherein in the first position, the at least one magnet is moved distally from a magnetic processing tube holder and wherein in the second position, the at least one magnet is moved adjacent to the magnetic processing tube holder. The precision pump may have at least one cylinder comprising a piston; a cylinder actuator rod; and an upper and lower port for fluidly connecting the cylinder to one or more pipettor assemblies.

The present invention provide an automated method for isolating DNA or RNA from a biological Lysate containing DNA or RNA, which has the three steps. First, one mixes a first reagent (a Lysis solution) having a pH of at least 7 into a sample and homogenizing the sample so that the cells in the sample are lysed to release nucleic acid from the sample to form a lysate, and mixing the Lysate with magnetic particles in glycerol in a magnetic processing tube. In this embodiment, the Lysis reagent contains (1) a lithium salt at a concentration of about 2-10 M; (2) a surfactant at a concentration of about 5-20% by volume; (3) a buffer; (4) a chelating agent at about 5-20 mM; (5) a detergent at about 0.05 - 5%; and (6) an antifoaming agent at about 0.005-0.1% by volume. Second, one optionally adds a second reagent to the lysate, such that DNA or RNA quantitatively bind to the magnetic particles; wherein the second reagent contains (1) a lithium salt at a concentration of at least 5 M; (2) a surfactant concentration of at least 5%; (3) a buffer; and (4) optionally, an alcohol such as ethanol or isopropanol, or the second reagent may be an alcohol such as ethanol or isopropanol. Third, one applies a magnetic force to the magnetic processing tube, wherein the DNA or RNA is liberated from the sample. In certain embodiments, the buffered binding solution contains a lithium salt at a concentration of at least 9 M, a surfactant at a concentration of at least 10%, and a pH of 8. In certain embodiments, the alcohol binding solution comprises lithium salt at a concentration of at least 5 M and an alcohol at a concentration of at least 55% v/v.

The present invention provides lysis solutions for lysing cells containing a lithium salt (*e.g*., lithium chloride or lithium bromide) at a concentration of about 2 M to about 10 M, a surfactant of about 5 % to about 40 %, a buffer (*e.g*., Tris), wherein the solutions have pH values of at least 7 to about 11, each containing a chelating agent of about 5 mM to 20 mM, each containing a detergent of about 0.05 % to about 2%, and each containing an antifoam agent (e.g., Dow Corning^{®} Antifoam A compound) of about 0.005 % to about 0.1%. In certain embodiments, the lysis solution may contain 20 mM TCEP. In certain embodiments, the surfactant is diethylene glycol monoethyl ether (DGME). In certain embodiments the buffer is Tris buffer. In certain embodiments, the solution has a pH of at least about 9, or even at least about 11. In certain embodiments the detergent is a non-ionic detergent, such as a Tween class detergent, a Triton class detergent, a Tergitol detergent, Nonidets or Igepal. In certain embodiments, the detergent is an anionic detergent, such as SDS (sodium dodecyl sulfate). In certain embodiments the surfactant is diethylene glycol monoethyl ether (DGME). In certain embodiments, the lithium salt is present at a concentration of about 2 M to about 5 M. In certain embodiments, the lysis solution contains 50 mM Tris (pH 10-11), 2 M LiCl, 0.1% SDS, 30% DGME, 10 mM citrate and 0.05% Dow Corning^{®} Antifoam A compound. In an alternative embodiment, the lysis solution contains 100 mM Tris (pH 7), 8 M LiCl, 0.1 % Triton X, 10% DGME, 10 mM EDTA and 0.01 % Dow Corning^{®} Antifoam A compound.

In certain embodiments the buffer is citrate buffer. In certain embodiments, the chelating agent is ethylenediaminetetraacetic acid (EDTA). In certain embodiments, the chelating agent is citrate. In certain embodiments, the detergent is a nonionic detergent, such as a Tween class detergent, a Triton class detergent, a Tergitol detergent, Nonidets or Igepal. In certain embodiments the detergent is an ionic detergent, such as the anionic detergent sodium dodecyl sulfate (SDS). In certain embodiments the antifoam agent is Dow Corning^{®} Antifoam A compound.

The present invention provides a reagent pack for purifying DNA, RNA, or both DNA and RNA, where the reagent pack has several separate compartments which contain purification reagents. In one embodiment for DNA, the reagent pack contains a lysis solution, a binding solution, one or more wash solutions and an elution solution. In another embodiment for DNA, the reagent pack contain one or more wash solutions and an elution solution. In one embodiment for RNA, the reagent pack contains one or more wash solutions, a DNase solution, an RNA rebinding solution, and an elution solution. In certain embodiments, the reagent pack further comprises a sealed cover, such as a pierceable material (*e.g*., a polypropylene cover) and/or a silicone evaporation barrier. The present invention provides a kit comprising packaging material and storage media for magnetic beads comprising glycerol, silica-coated magnetic particles, buffer, and in certain embodiments Proteinase K.

Other features and advantages of the invention will be apparent from the following detailed description, and from the claims. Unless otherwise defined, all technical and scientific terms used herein have the meaning commonly understood by one of ordinary skill in the art to which this invention belongs. All publications, patent applications, patents, and other references mentioned herein are incorporated by reference in their entirety. In case of conflict, the present specification, including definitions, will control. The disclosed materials, methods, and examples are illustrative only and not intended to be limiting. Skilled artisans will appreciate that methods and materials similar or equivalent to those described herein can be used to practice the invention.

### BRIEF DESCRIPTION OF THE FIGURES

Figure 1 shows a schematic of a top view (A), a side vie (B) and a perspective view (C) of a Process Assembly that can be used in the method of the present invention.
Figure 2 shows a schematic of a Process Assembly (A), a Magnetic Processing Assembly (B), and a Magnetic Elevator Assembly (C), that can be used in the method of the present invention.
Figure 3 shows a schematic of an apparatus for isolating nucleic acid as described herein.
Figure 4 shows a schematic of an Upper Deck Assembly and a Pump Assembly.
Figure 5 shows a schematic of a single cylinder from a Pump Assembly. Top Port 69 connects to valve which connects to Dispense Pipettor Assembly 48. Bottom Port 70 connects to valve which connects to Aspiration Pipettor Assembly 38.
Figure 6 shows a schematic of one embodiment of an Electronic Control System, that can be used in the method of the present invention.
Figure 7A shows a Reagent Pack, and Figure 7B shows an exemplary Reagent Pack Holder holding a series of Reagent Packs that could be used for a direct lysis protocol described herein.
Figure 8A shows RNA samples analyzed by gel electrophoresis. The sample was treated following the RNA Lysate Protocol. 5 Million K562 Cells were used with or without a Binding Solution addition step. Figure 8B shows RNA Lysate Protocol yield per million Cells. One and five 5 Million K562 Cells were used with or without Binding Solution addition step.
Figure 9A shows a DNA Lysate electrophoresis gel. Five million cultured cells were treated with or without RNase A. Figure 9B is a DNA Lysate yield graph. Five million cultured cells were used with or without RNase A treatment.

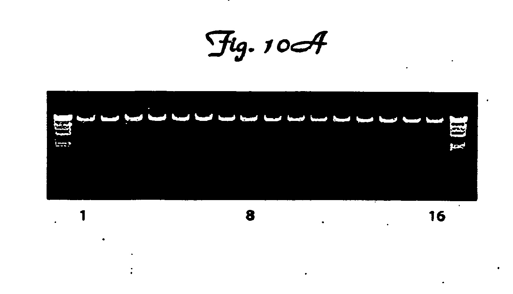

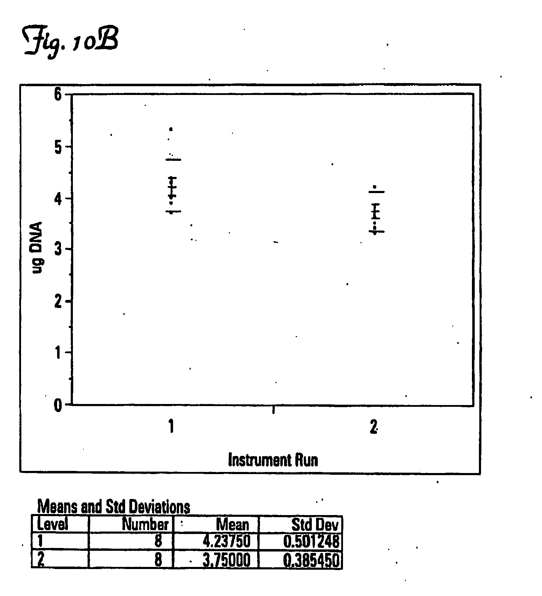

Figure 10A shows a DNA Direct Lysis electrophoresis gel. Two hundred µL Whole Blood was tested in two instrument runs of eight samples each. Figure 9B is a DNA Direct Lysis yield graph. A two hundred µL whole blood sample was used in each of two instrument runs of eight samples each.

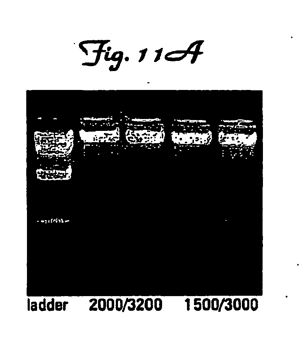

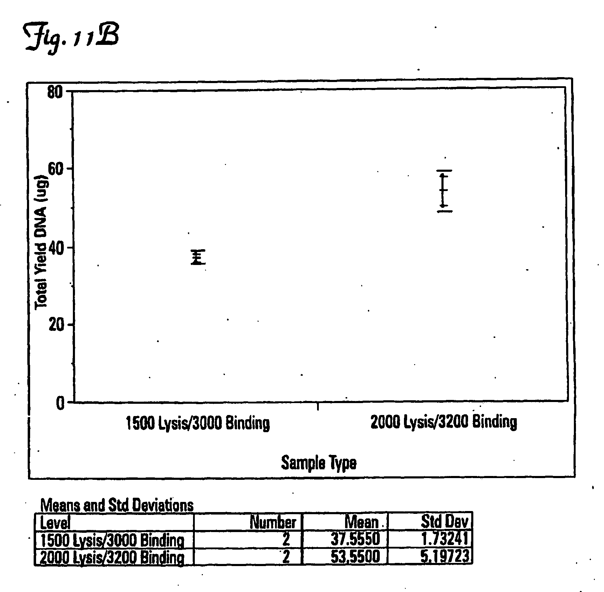

Figure 11A shows a DNA Direct Lysis electrophoresis gel. Five hundred µL Buffy Coat Lysis was tested and Binding Volume Variation. Figure 11B shows a DNA Direct Lysis yield graph. Five hundred µL, Buffy Coat was used. The graph shows lysis and binding volume variation.

### DETAILED DESCRIPTION

Developments in the biological, medical and pharmacological sciences have increased the interest in studying genes, and have intensified the need for sophisticated methods to obtain nucleic acids from a variety of samples. For example, ribonucleic acids provide extensive information of the genetic origin and the functional activity of cells. Such information can be used, for example, in clinical practice, to diagnose infections, detect the presence of cells expressing oncogenes, detect heredity disorders, monitor the state of host defense mechanisms, investigate and diagnose metabolic diseases, investigate influence of drugs on gene expression in patients, and investigate side and toxic effects of drugs.

Numerous nucleic acid purification methods exist that fall into two general categories, liquid phase purification and solid phase purification. In liquid phase purification, nucleic acids remain in the liquid phase, while impurities are removed by precipitation and/or centrifugation. Alternatively, nucleic acids are precipitated out of solution while the impurities remain. In solid phase purification, the nucleic acids are bound to a solid support, while impurities are selectively eluted. For example, RNA isolated by liquid phase purification remains in the liquid phase, while impurities are removed by processes such as precipitation and/or centrifugation. In solid phase purification, RNA is bound to a solid support while impurities such as DNA, proteins, and phospholipids are selectively eluted. Both purification strategies utilize conventional methods, which require numerous steps and may use hazardous reagents, as well as more rapid methods, which require fewer steps and usually less hazardous reagents. In the case of DNA and RNA purifications, if the starting material (*e.g*., biological material) includes cells, both the liquid and solid phase methods require rupturing of the cells, or a lysis step. A lysis step results in DNA and RNA mixed with contaminants such as protein, lipids, carbohydrates, etc. Such a mixture also contains DNases and RNases that degrade DNA or RNA and must be removed and/or inactivated, so as to not interfere with yielding substantially undegraded DNA or RNA.

### Instrument Components

Numerous instruments exist that allow a user to obtain nucleic acid from a sample. For example, many commercially available liquid handler systems have modular stations that move liquids around the work station to automate routine liquid-handling tasks and nucleic acid purification for general molecular biology research. Such instruments generally contain pipettes for aspiration, sensing mechanisms for fluid levels, and other robotics for moving the sample and fluids to specified areas along the work station. See, *e.g*., U.S. Patent Nos. 5,443,791 and 6,060,022. These work stations may further contain analysis functions to perform diagnostic assays (see, *e.g*., U.S. Patent No. 6,605,213). Some commercially available instruments for isolating nucleic acids include, for example, liquid handlers such as those sold under the Tecan™ and Perkin-Elmer brands. Other instruments for nucleic acid isolation are sold under the brands BioRobot™ (Qiagen AG), MagNA Pure™ (Roche), Autopure LS™ (Gentra Systems, Inc.), and Autogenprep™ (AutoGen). Most of these instruments are large and bulky, taking up twenty or more square feet of laboratory space, as well as being very expensive.

Recently, a few companies have developed smaller, more compact instruments for nucleic acid purification. For example, the MagNA Pure Compact™ (Roche Applied Science) is for low-throughput (1-8 isolations per run) nucleic acid purification. The features of the instrument include pre-filled single-use reagent cartridges and a bar code reader. Also available is the EZI™ (Qiagen). The Roche and Qiagen instruments are identical in their methods of isolating and purifying DNA in that they use magnetic particles and chaotropic reagents such as guanidine. Because of the single-use consumables as well as the internal components of these instruments, buying and using these instruments for nucleic acid isolation can be expensive ($25,000 to purchase the instrument plus $6.00-$10 per sample thereafter).

A compact and economical apparatus that can be used in the methods of the present invention for includes as isolating nucleic acids (*e.g*., DNA and RNA) from a biological sample basic components but is not limited to multi-use Reagent Packs 24 and other disposables, a Process Assembly 10, a Magnetic Elevator Assembly 28, an Upper Deck Assembly 56, and an Electronic Control System 78 with a User Interface 82, and pipettors (liquid handler assemblies).

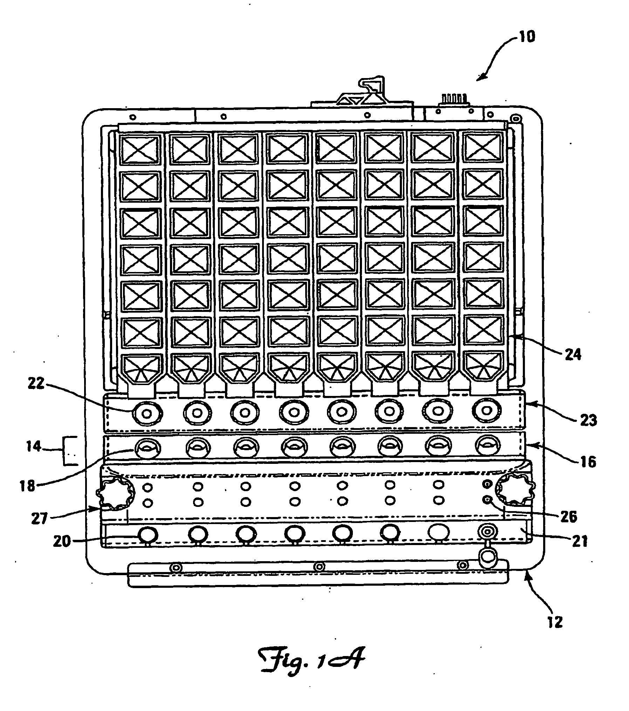

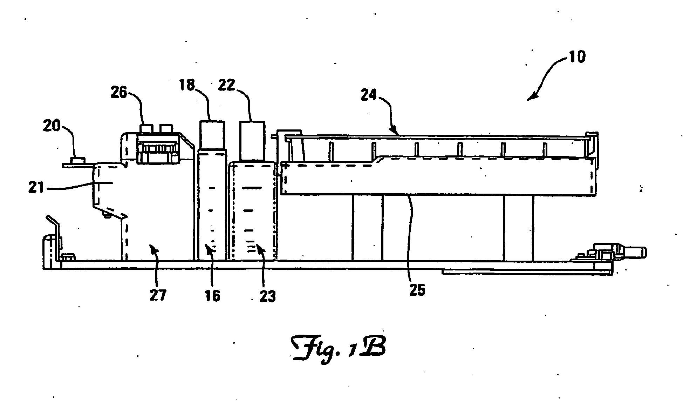

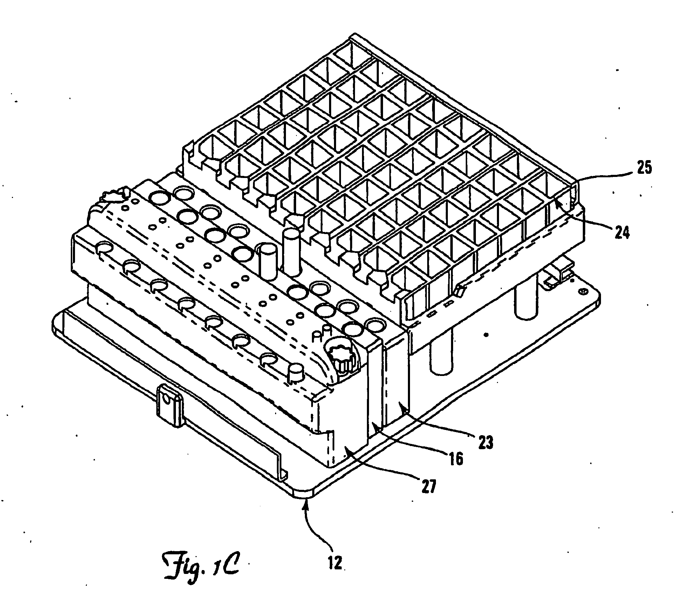

Figures 1A, 1B and 1C show a top-view, a side-view and a perspective-view, respectively, of the Process Assembly 10, and other components. The Process Assembly 10 is where disposables and reagents are positioned. The Process Assembly 10 comprises a Mounting Plate 12 that can slide partially out of the instrument for loading and completely out of the instrument for cleaning. The Mounting Plate 12 includes a Magnetic Processing Assembly 14 with Magnetic Processing Tube Holder 16 that holds Magnetic Processing Tubes 18, as well as an Output Tube Holder 21 that holds Output Tubes 20, and Waste Tube Holder 23 that holds Waste Tubes 22, Reagent Pack Holders 25 that hold Reagent Packs 24, and Disposable Pipette Tip Holder 27 that holds Disposable Pipette Tips 26. The Process Assembly 10 can contain the disposables (*e.g*., tubes, tips, and Reagent Packs) used in the nucleic acid isolation process, and provides fluid ingress protection for susceptible components. The disposables (*e.g*., tubes, tips, and Reagent Packs) are located in rows, and in one embodiment, can be present in a mounting plate in the following order (from the front to the back of the mounting plate): 1 row of 1-8 Output Tubes 20; 2 rows of 1-8 Disposable Tips 26;1 row of 8 Magnetic Processing Tubes 18 containing particles; 1 row of 1-8 Waste Tubes 22, and 1 row of 1- 8 Reagent Packs 24. The order of instrument disposables can vary in different embodiments. Examples of tubes that can be used for the Magnetic Processing Tubes 18 and Waste Tubes 22 include, for example, 10 mL capped conical bottom tubes (Sarstedt). Examples of tubes that can be used for the Output Tubes 20 include, for example, 1.5 mL Eppendorf centrifuge tubes.

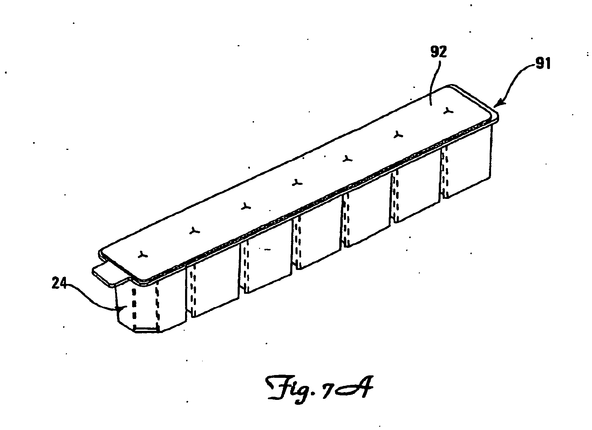

There may be a number of different reagents used in nucleic acid isolation. As described herein, a Mounting Plate 12 contains an area for reagents. Different reagents or a combination of reagents are used depending upon the nucleic acid isolation protocol. A reagent or a combination of reagents can be formulated into a Reagent Pack 24 and positioned appropriately in a Mounting Plate 12. The number of Reagent Packs 24 may vary from one to eight, corresponding to the number of samples to be processed. Reagent Packs 24 can have seven wells that are used for isolating DNA. The wells may contain Lysing Solution, Binding Solution for use with the direct lysis method, one or more wash solutions, an elution solution, and other auxiliary reagents, such as DNase reagents; for example when isolating RNA. A Reagent Pack 24 can contain cleaning solutions for maintaining the instrument and/or apparatus. The number of wells in a Reagent Pack 24 depends on the number of reagents desired. An example of a Reagent Pack 24 is shown in Figure 7A. A Reagent Pack 24 can be made using any number of materials. The reagent(s) may be accessed by piercing the Primary Seal 91 and the Secondary Seal 92 of the Reagent Pack 24 with at least one pipette tip. The Primary Seal 91 and/or the Secondary Seal 92 can be made of a re-sealable material to avoid contamination and/or evaporation of the reagents contained in the Reagent Pack 24. The reagents may also be accessed through a septum or a port in the Reagent Pack 24. Multiple Reagent Packs 24 can be positioned in the Mounting Plate 12.

The Mounting Plate 12 of the Process Assembly 10 has a Disposable Tip Holder 27 such that an Aspiration Pipettor Assembly 38 can automatically load the Disposable Tips 26. This automation eliminates the labor-intensive work of loading tips as well as potential contamination of the tips by the user. All the tips generally are loaded simultaneously. The Aspiration Pipette Assembly 38 does this by moving into position above the Disposable Tip Holder 27, and moving down onto the Disposable Tips 26 while applying force.

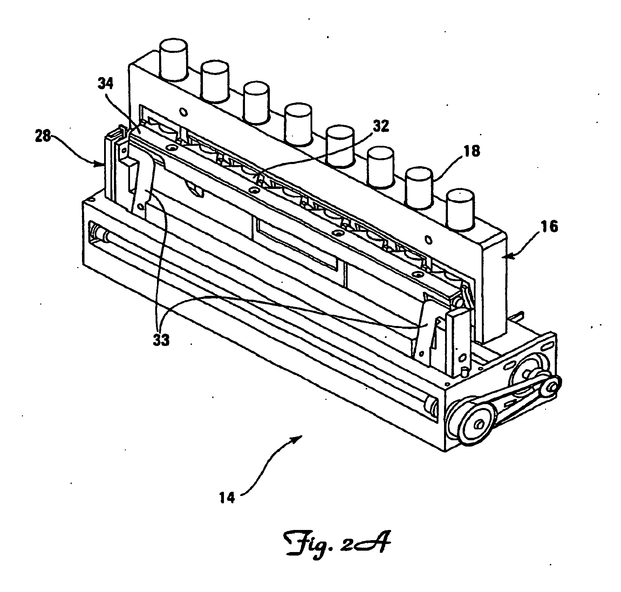

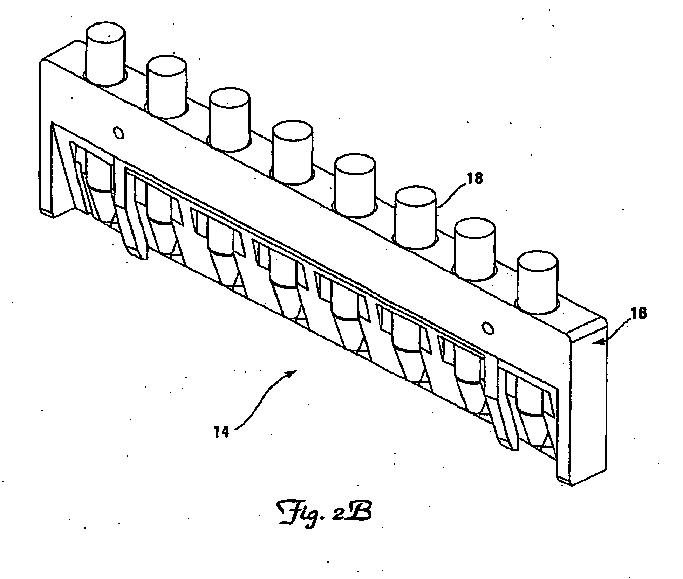

Figure 2B shows a Magnetic Processing Assembly 14 that can be used in the methods of the present invention, where much of the processing of the samples is performed using magnetic particles. The Magnetic Processing Assembly 14 contains a Magnetic Processing Tube Holder 16 that holds the Magnetic Processing Tubes 18 and allows for Magnets 32 in the Magnetic Elevator Assembly 28 to move into very close proximity (within 0.032") to the wall of the Magnetic Processing Tube 18. The Magnetic Elevator Assembly which can include, a pivoting Magnet Holder 34 and/or Springs 33, is moved using a drive system. The drive system can include a pivoting Magnet Holder 34 and/or Springs 33. The Magnets 32 typically follow the curves of a sample tube (*e.g*., a conical tube). The Magnetic Processing Tube Holder 16 can have an integral heater that can heat the sample to, for example, 45 ± 2°C. The heater is turned on and off as needed for DNA processing, and usually is not used for RNA processing. The Magnetic Processing Tubes 18 can come in contact with the heated wall of the Magnetic Processing Holder 16 by for example, moving along the wall at a range from the bottom of the tube up to about the 8 mL fill line of a 10 mL tube.

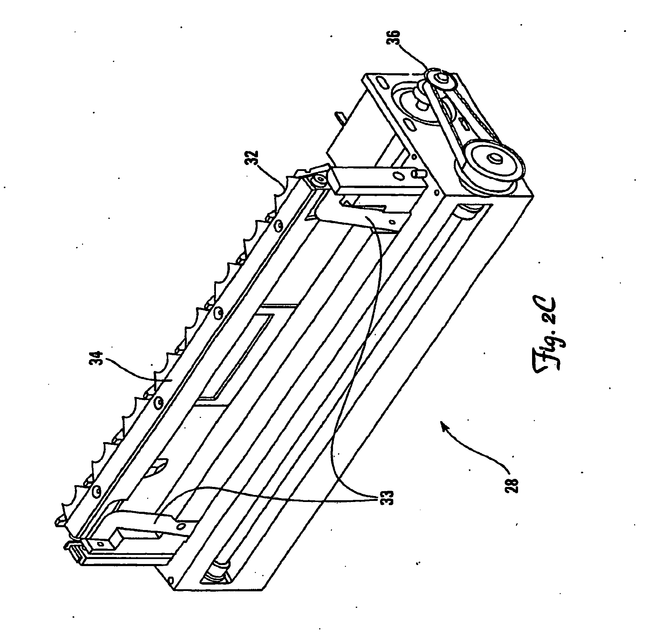

Figure 2C shows a Magnetic Elevator Assembly 28 that can be used in the method of the present invention that provides a novel means to optimally position Magnets 32 (for capturing the magnetic particles) relative to the volume of fluid being processed. A Magnetic Elevator Assembly 28 consists of a Magnet Mounting Assembly 34 and a Drive System 36. The Magnet Mounting Assembly 34 mounts, for example, eight Magnets 32 in positions corresponding to the locations of the eight Magnetic Processing Tubes 18. Such Magnets 32 can be made from, for example, neodymium iron boron (NdFeB).

The Magnetic Elevator Assembly 28 is capable of positioning Magnets 32 anywhere between the "top of travel" and "home" positions during processing by way of a pivot point on the assembly. The travel velocity of a Magnetic Elevator Assembly 28 typically is 0 inches/sec to 1.5 inches/sec. For example, the "top of travel" position as used herein can refer to the top of the Magnet 32 being at or above the 6.0 mL fill-line on a 10 mL tube (or for example 2/3 from the top of tube, depending on the size of the tube. The "home" position as used herein can refer to the top of magnet being just below the bottom of the tube.

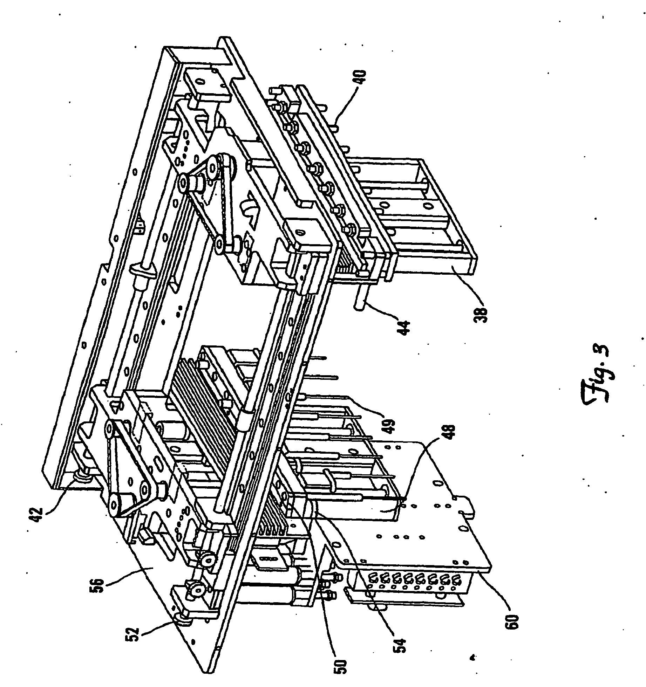

Figure 3 is a schematic of the Aspiration Pipettor Assembly 38 and Dispense Pipettor Assembly 48 that can be used in the method of the present invention. The Aspiration Pipettor Assembly 38 is used to manipulate samples and reagents within the Magnetic Processing Tubes 18, deliver waste from the Magnetic Processing Tubes 18 to the Waste Tubes 22, and to deliver purified nucleic acid to the Output Tubes 20. An Aspiration Pipettor Assembly 38 performs these functions in conjunction with a Pump Assembly 60. The Aspiration Pipettor Assembly 38 is also capable of automatically loading and unloading the Disposable Tips 26 used in processing samples and detecting sample levels. The Aspiration Pipettor Assembly 38 may consist of a Tip Mounting System 40 for connecting to Disposable Tips 26, an Aspiration Drive System 42 for positioning the tip mount vertically, and a Tip Ejection System 44, and may contain an Aspiration Fluid Level Sense Circuitry 46. The Aspiration Pipettor Assembly 38 can pick up Disposable Tips 26 from the appropriate position on a Tip Holder 27 of a Process Assembly 10. The Aspiration Pipettor Assembly 38 can automatically eject tips, for example, into a Waste Tube 22. An Aspiration Pipettor Assembly 38 can receive from one to eight tips. Additional rows can be added to the mounting plate to increase the number of tubes and tips, thus increasing the number of samples run by the apparatus. The tips that can be used include, for example, without limitation, Eppendorf disposable tips. In some embodiments, the Tip Mounting System 40 can be spring loaded to allow for installation of a pipette tip with controlled force.

Generally, an Aspiration Pipettor Assembly 38 is positioned at the rear of the apparatus when a user opens the door or requests that the doors open. In certain embodiments, when the doors of an apparatus as described herein are closed, an Aspiration Pipettor Assembly can resume its position prior to interruption. The Aspiration Pipettor 38 can be equipped with an Aspiration Fluid Level Sense Circuitry 46 to determine the presence and volume of a sample.

Figure 3 also shows a schematic of the Dispense Pipettor Assembly 48 used to deliver reagents from on-board Reagent Packs 24 to the Magnetic Processing Tubes 18. A Dispense Pipettor Assembly 48 performs these functions in conjunction with a Pump Assembly 60. The Dispense Pipettor Assembly 48 consists of a Dispense-Tip Mounting System 50 for mounting tips, generally Fixed Tips 49 (*i.e*., non-disposable tips), a Dispense Drive System 52 for positioning the tip mount vertically, and Dispense Fluid Level Sense Circuitry 54. The Dispense Pipettor Assembly 48 can be used to actuate the Tip Ejection System 44 on the Aspiration Pipettor Assembly 38.

The following specifications are representative for a Dispense Pipettor Assembly 48 described herein. The Home Z Position, which essentially equals the "top of travel," means that the fixed tip must clear the highest obstacle (*e.g*., the top of the highest tube) by greater than 0.1 inches. The positional accuracy requirement of a Dispense Pipettor Assembly is ± 1.27 µm (0.005 inches). The system described herein is capable of positioning the pipette tips at any position between the Home Z Position and the max extension (*i.e*., the bottom of travel) during processing. The total travel distance may be greater than or equal to 10.4 cm (4.1 inches), and a representative travel velocity requirement is, for example, 0 cm/s (0 inches/second) to 6.35 cm/s (2.5 inches/second). A Dispense Pipettor Assembly 48 as described herein can include a mechanism to determine when the Assembly is at the Home Z Position. The fixed tip end location may have a tolerance of within 813 µm (0.032) inches in diameter.

The Dispense Pipettor Assembly 48 can have up to eight Fixed Tips 49, each with a capacity of 1000 µl, attached to the Dispense Tip Mounting System. The Dispense Pipettor Assembly 48 can be equipped with a fluid level sensing circuitry to verify the level of all reagents before a procedure starts. The apparatus as described herein minimizes the possibility of reagent-to-reagent cross contamination. In addition, the apparatus described herein can be capable of cleaning and decontamination using decontamination reagents in, for example, a Reagent Pack 24.

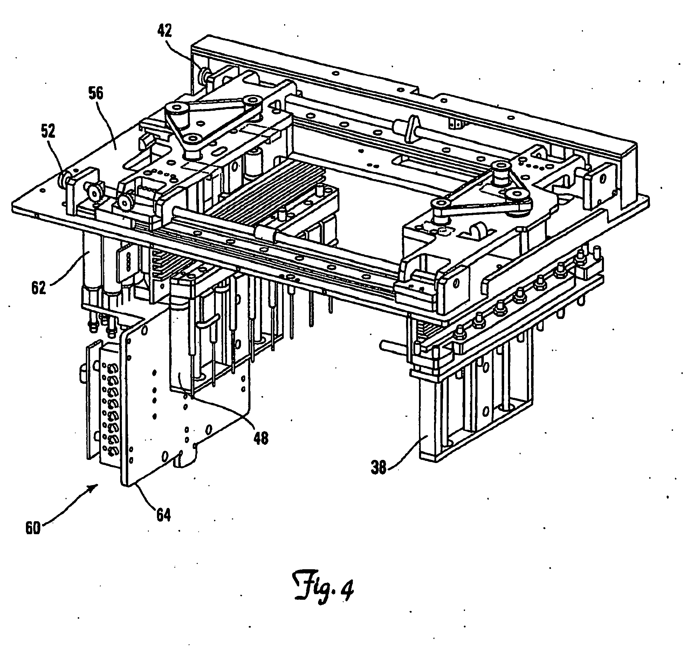

Figure 4 shows a schematic of an Upper Deck Assembly 56, which contains one or more "Y" direction drive mechanisms 42, 52 to manipulate the pipettes, as well as components for managing cables and tubing. The drive mechanism(s) positions the Pipettor Assemblies so that they may access the appropriate Reagent Pack, tube, or tip. The Upper Deck Assembly 56 typically has a mechanism that limits the movement of the Aspiration Pipettor Assembly 38 to ensure that the Aspiration Pipettor Assembly 38 cannot reach a Reagent Pack 24. An Upper Deck Assembly 56 may include an instrument to detect when an Aspiration Pipettor Assembly 38 is in the Home Y position. The Home Y Position is when an Aspiration Pipettor Assembly is moved to a rearward position (*e.g*., farthest from operator). Since both the Aspiration 38 and Dispense 48 Pipettor Assemblies must access the Magnetic Processing Tubes 18 individually, system software can ensure that no collisions take place between the Assemblies. Also, for safety, the Aspiration Pipettor Assembly 38 can retract to a rearward position any time the access doors are opened.

Figure 4 also shows a Pump Assembly 60 in conjunction with the Upper Deck Assembly 56. The Pump Assembly 60 is used to pick up and deliver reagents from the Reagent Packs 24, as well as to mix samples with reagents, remove waste, and deliver purified nucleic acids to the Output Tubes 20. The Pump Assembly 60 typically consists of eight standard Double-acting Air Cylinders 62, a Base Structure 64, and a Motor 68 (*e.g*., a stepper motor). Correspondingly-numbered Dispense Fixed Tips 49 and Disposable Tips 26 share an air cylinder. The tips for dispensing reagents are connected to the "open" end of the cylinders via the Dispense Pipettor Assembly 48, and the tips for aspiration are connected to the ends of the cylinders containing Cylinder Actuator Rods 74 via the Aspiration Pipettor Assembly 38.

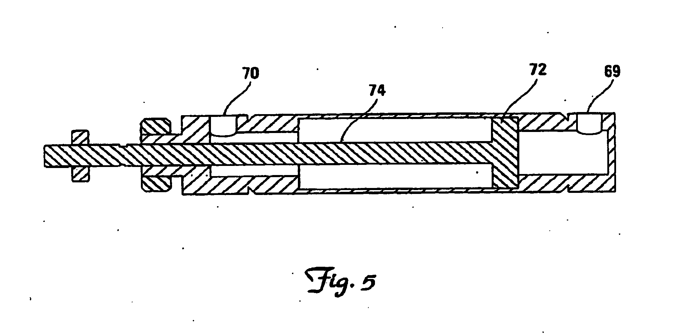

The Pump Assembly 60, for example, uses eight cylinders and one motor to provide sixteen channels of fluid control. Figure 5 shows a schematic of an individual cylinder within a Pump Assembly 60 as described herein. Each Double-acting Air Cylinder 62 has two connections; a Top Port 68 and a Bottom Port 70. The Top Ports 68 are connected via a valve to the back (Dispense) pipettor (total of eight channels), and the Bottom Ports 70 are connected via a valve to the front (Aspiration) pipettor (eight more channels). The Double-acting Air Cylinders 62 have Pistons 72 inside that sit at a mid-point between the two outlet ports. When pumping to and from the back pipettor is required, all eight pistons move inside the Double-acting Air Cylinders 62 and the valves that are between the cylinder and the pipettor are opened. In other words, the Double-acting Air Cylinders 62 only move if there is a sample present (*i.e*., the number of moving cylinders is equal to the number of samples in the run). The valves to the front pipettor are switched to vent, which effectively isolates the front pipettor from the back pipettor. When the front pipettor is to be used, the situation is reversed, allowing the back pipettor to be isolated. In essence, one pump that serves two different pipettors is being used instead to two independent pumps. In one embodiment the "front" pipettor is the Aspiration Pipettor Assembly 38 and the "back" pipettor is the Dispense Pipettor Assembly 48.

The Pump Assembly 60 connects to the Dispense Pipettor Assembly 48 to dispense reagents required for the purification process. The apparatus draws reagents from the Reagent Packs 24 into the fixed dispense tips and the connected tubing. The Pump Assembly 60 also connects to the Aspiration Pipettor Assembly 38 to mix and redistribute samples and reagents. The apparatus draws the samples from the Magnetic Process Tubes 18 into the disposable tips (*e.g*., disposable, filtered tips). The Pump Assembly 60 can have a travel sensor and can verify its location after every dispensing activity.

Each connection to each pipette tip in a Pipettor Assembly has a Valve Convector 76. This Valve Connector 76 is controlled by software to close it a tip is not being used. Each Valve Connector 76 can be connected to an electronic circuit that monitors the current used to determine the proper operation of the valve. Each channel of the pump can be equipped with a pressure transducer to verify the pressure change due to aspiration and dispense. The materials and fittings can be made out of materials that are compatible with the reagents used in the apparatus, including cleaning fluids (*e.g*., bleach).

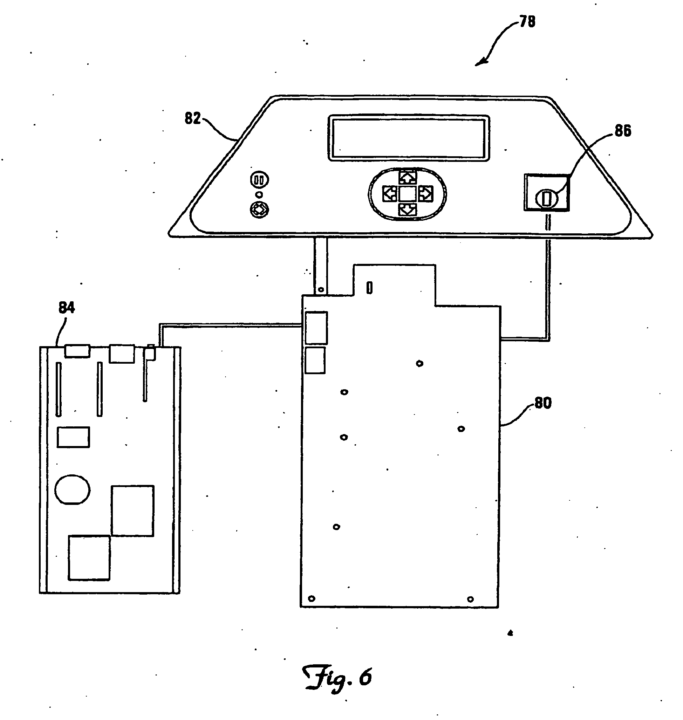

Figure 6 shows an Electronic Control System 78 that includes a programmable Microcontroller 80, a User Interface 82 with display, a Universal Power Converter 84, and a Dedicated Data I/O port 86. The Electronic Control System 78 also can have, for example, an eight channel level sensor, a power management system, a heater, and/or a six motion control channel. Proprietary control software can be used to implement all electronic and fluidic features of this instrument. The Electronic Control System 78 allows for detecting the level of fluid via, for example, conductive dispensing tips. Representative conductive dispensing tips can sense in 8 x 3 discrete sample/process positions and 2 x 6 reagent positions. Data input and output can come, for example, via a proprietary portable DATAKEY type "memory stick."

The primary system control functions may be managed by a Fujitsu F²MC-16LX microcontroller. This 16-bit microcontroller operates at 16-megahertz, has 128K bytes of programmable flash memory, 81 general purpose control ports, 2 serial communication ports, and 8 A/D converters.

The User Interface 82 generally consists of a monochrome display, a membrane switch keypad, a data transfer device, and an audible alarm. For example, a display can use Vacuum Fluorescent Technology and have a display area of 115 mm x 28 mm. Elements of the User Interface 82 are water and splash proof, and resistant to common lab equipment cleaning chemicals. A User Interface 82 typically includes a data transfer device such as a memory chip that is used to download system software updates and new protocols into the instrument. Protocols may include for example extraction of DNA and/or RNA from blood, tissue, cells, plants, bacteria, and other samples described herein below. The User Interface 82 generally includes a "pause" key that stops the protocol normally and allows for restarting of a protocol. The elements of a User Interface 82 can be mounted to an enclosure panel in the front of the apparatus, and generally are the primary control point for the operation of the instrument.

Motors (*e.g*., stepper motors) can be independently controlled by the Microprocessor 80 using individual motion controllers. A heater can heat samples using an insulated resistive heating element controlled by dedicated circuitry. For example, the maximal attainable temperature can be set at 50°C and the heating element can have an appropriate thermal cut-off. The system also can have a sleep-mode power saving feature when not in use.

The apparatus can be constructed, for example, inside a frame assembly that supports and positions the major elements of the instrument. A frame assembly can consist, for example, of a housing and a power assembly. The housing and/or the power assembly can provide a structure in which to mount the system power supply and the power components (*e.g*., power inlet module, primary power switch, power supply, and/or cooling fan). The left and right panels of a frame assembly can support the Upper Deck Assembly 56 and allow for adjustment so that the Upper Deck Assembly 56 is parallel to the Process Assembly 10. The rear panel of the frame assembly can support the fluid handling components, the Pump Assembly 60, the Aspiration drive System 42, and the Dispense Drive System 52.

The frame-assembly can be enclosed by any combination of fixed skins, removable panels (*e.g*., for service access), and/or a hinged door (*e.g*., for user access). The primary purpose of the enclosure components of the frame assembly is to protect the operator from contact with moving parts and electrical devices. In addition, spill containment can be addressed in the design of the frame assembly and means of enclosure.

### Samples

Samples, such as biological samples, can be collected by a variety of means. For example, sample collection containers are used for collecting and storing samples. In some embodiments, collection containers are glass or plastic tubes having a resilient stopper. In other embodiments, blood collection tubes are used, where the tube is evacuated to draw a volume of blood into the tube. In some embodiments, collection tubes can have various additives, such as ethylenediaminetetraacetic acid (EDTA) contained therein, in order to prepare the blood sample for a particular test. In certain embodiments, samples are biological fluids (*e.g*., whole blood, bone morrow, blood spots, blood serum, blood plasma, buffy coat preparations, saliva and cerebrospinal fluid, buccal swabs, cultured cells, cell suspensions of bacteria, solid animal tissues such as heart, liver and brain, body waste products, such as feces and urine, environmental samples taken from air, water, sediment or soil, plant tissues, yeasts, bacteria, viruses, mycoplasmas, fungi, protozoa, rickettsia, and other small microbial cells). In other embodiments, samples are lysates, homogenates, or partially purified samples of biological materials. In other instances, biological materials include crude or partially purified mixtures of nucleic acids.

### Instrument Consumables.

The instrument that can be used in the method of the present invention uses consumables such as reagents are contained in Reagent Packs 24, a solid support such as magnetic particles contained within Magnetic Processing. Tubes 18, Waste Tubes 22, Output Tubes 20, and Disposable Pipette Tips 26.

Reagent Packs 24 are trays with a variety of compartments for holding reagents. The trays may be made from plastic or other suitable materials. The Reagent Packs 24 are sealed using methods known in the art such as adhesives, heat sealing or ultra sonic welding. The Reagent Pack 24 may be sealed with polypropylene or with a silicone evaporation barrier. Reagent Packs 24 may also have two seals, a Primary Seal and a Secondary Seal 92 (Figure 7A). The Primary Sepal 91 may be a laminate of polyester and polypropylene. The Secondary Seal 92 may be positioned on top of the Primary Seal 91 and may be made of a self-sealing material such as silicone. This permits the reagent pack to be pierced repeatedly such that evaporation is prevented and reagent stability maintained allowing the reagent pack to be used for multiple purifications over an extended time period. The Reagent Packs 24 hold sufficient volumes of reagents for multiple reactions. For example, a Reagent Pack 24 may hold sufficient reagents for one to eight purifications. In other nucleic acid purification instruments, the Reagent Packs 24 are single-use and therefore are costly. The Reagent Packs 24 can contain, Lysis Solution, an optional Binding Solution, one or more Wash Solutions, an Elution Solution, and auxiliary reagents necessary for purification of nucleic acid as well as for cleaning and maintenance of the instrument.

Another consumable component of the instrument is the Magnetic Processing Tubes 18. The tubes contain solid supports, and may contain packaging reagents. A packaging reagent is a reagent that allows the solid support to be contained within the Magnetic Processing Tube 18 during shipment.

Solid supports are used in the present invention. Suitable solid supports are silica magnetic particles. In some embodiments, the solid support may be encased or immobilized in a vessel such as a tube. In some embodiments, the solid support is a plurality of paramagnetic particles. In some embodiments, the paramagnetic particles are made of an iron oxide (ferric oxide, magnetite) core and are coated with silica dioxide. Silica dioxide produces a hydrophilic surface with terminal Si-OH bonds. The particles may have a size distribution of 2-10 µm. In some embodiments, the particles have a size distribution between 5.0 and 8.5 µm, such as about 6 µm. The particles may have a pore size of greater than 500 angstroms. In some embodiments, the pore size is 600 to 700 angstroms. In some embodiments, the particles may be nonporous. Suitable particles include, for example, MagneSil® particles (Promega) or MP-50 Magnetic Substrates (W.R. Grace).

Acceptable packaging reagents for the magnetic particles include a gel, a polymeric fluid, or a viscous liquid, which keeps the particles contained in the bottom of the magnetic processing tube, such that they do not flow and splash substantially within the tube during transport and use. In the 200 µL DNA direct lysis whole blood protocol described below the magnetic particle suspension is processed by adding 6 - 9 µL of a 20 mg/mL Proteinase K solution, 46 µL of buffered particle suspension in water (Promega), and 11 µL of glycerol in the bottom of a 10 mL conical tube. The tube is dried in an oven to remove water, without denaturing the Proteinase K, resulting in a suspension of magnetic particles, Proteinase K and buffet in glycerol. In the 500 µL DNA direct lysis whole blood, 200 µL DNA buffy coat, and 500 µL DNA buffy coat protocols described below, the magnetic particle suspension is processed by adding 13 -17 µL of a 20 mg/mL Proteinase K solution, 130 µL of buffered particle suspension in water (Promega), and 28 µL of glycerol in the bottom of a 10 mL conical tube. The tube is also dried in an oven to remove water, resulting in a suspension of magnetic particles, Proteinase K and buffer in glycerol. In the DNA Lysate and RNA Lysate protocols described below the magnetic particle suspension is processed by adding 130 µL of buffered particle suspension in water (Promega), and 33 µL of glycerol in the bottom of a 10 mL conical tube. The tube is also dried in an oven to remove water, resulting in a suspension of magnetic particles and buffer in glycerol.

Other magnetic particle packaging methods may be used. The particles may be in solution, in a polymer or natural film or in a dried form as well. In some instances, the particles are in a water solution. In some instances the particles are lyophilized. In some instances, the particles are mixed with a forming reagent and dried into a film or formed into a sphere. In some instances, the particles may be pre-treated with or associated with enzymes. In some instances, the particle solution may contain an RNase solution in order to degrade RNA present in the sample (*e.g*., a biological sample).

### Reagents

The present invention features Lysis solutions and Binding and Rebinding solutions. Wash Solutions and Elution Solutions may also be used.

### 1. Lysis Solutions

A Lysis Solution enables efficient lysis (*e.g*., of cells in a biological sample) to release nucleic acids, effectively inhibits nucleic acid degrading enzymes' activity, and in certain embodiments allows nucleic acids to quantitatively bind to a solid support of choice. A Lysis Solution of the present invention contains a buffer (such as Tris and/or citrate), an alkali metal salt (such as lithium salts, for example lithium chloride), a detergent (such as Triton or SDS); a surfactant (such as DGME), a chelator (such as EDTA or citrate), and an antifoaming agent (such as Dow Coming^{®} Antifoam A compound). The Lysis solutions-of the present invention are unique in that they require no added chaotropic salts, such as guanidinium salts or urea. Guanidinium salts and urea are strong chaotropic compounds that tend to disrupt the structure of water and tend to decrease the stability of hydrophobic interactions of compounds in solution. Guanidinium salts and urea dissolve in water through endothermic reactions. Both guanidinium salts and urea are considered to be strongly chaotropic salts as defined by the Hofmeister series, a widely used system that ranks cations and anions according to relative chaotropic strength (F. Hofmeister, On the understanding of the effects of salts, Arch. Exp. Pathol. Pharmakol. (Leipzig) 24 (1888) 247-260). The present invention involves the use of lithium salts, including for example, lithium chloride and lithium bromide. The lithium ion is considered very kosmotropic, due to its high surface charge density and strong hydration characteristics. The lithium ion is unique in that it has a small radius and therefore a high charge density. The larger surface charge density is responsible for.the tremendous interaction of the lithium ion with water molecules. Kosmotropes such as the lithium ion tend to organize water molecules around themselves and disrupt the solution stability of polar molecules, such as nucleic acids, which are stabilized by water molecules in solution. The solubilization reaction of lithium salts in water is a highly exothermic reaction and is indicative of the tremendous ion-dipole interaction exhibited by the strong kosmotropic lithium ion with water. Lithium salts, such as lithium chloride and lithium bromide, are highly soluble in aqueous solutions due to the ion-dipole interaction of the lithium ion and the resulting exothermic heart of solution for these salts upon solubilization in water.

A first component of the Lysis Solution is a buffer that maintains the pH of the solution (*e.g*., a Tris buffer or any known buffer). For example, the pH of the buffer may be at least about 7, at least about 8, at least about 9, at least about 10, or at least about 11, 11.5 or 12 (*e.g*., 6.8, 6.9, 7.0, 7.2, 7.3, 7.4, 7.5, 7.6, 7.8, 8.0, 8.1, 8.4, 8.6, 8.7, 8.9, 9.1, 9.5, 9.8, 10.1, 10.3, 10.5, 10.7, 10.9, 11.1, 11.2, 11.3,11.4,11.5,11.8,12,12.1,12.3 or 12.5). The optimal pH of a Lysis solution will differ depending on whether the operator is performing a DNA Direct Lysis procedure, or is performing a DNA or RNA Lysate procedure. In a DNA Direct Lysis procedure, the pH is buffered at a pH of greater than about 10, whereas with a Lysate procedure, the pH is buffered at a pH of about 7. The Tris may be used at a concentration of 50 - 150 mM (*e.g*., 60 mM, 70 mM, 80 mM, 90 mM, 100 mM, 120 mM, or 140 mM). In some embodiments, Tris buffer is an appropriate buffer. In some embodiments, for example for the DNA Direct Lysis protocol; Tris buffer is formulated with a pH of 11 and a concentration of 50 mM Tris base. In other embodiments, for example, the DNA and RNA Lysate protocols, a Tris buffer with a pH of 7 and a total acid and base concentration of 100 mM is used.

Another component of a Lysis solution is a lithium salt, or "complexing salt" (such as lithium salts; for example lithium chloride) that confers unique binding properties to nucleic acids (*e.g*., an RNA or DNA complexing salt). The nucleic acids are charge-neutralized by the lithium ions binding to the phosphate groups thereby decreasing their stability in solution such that the nucleic acids preferentially bind to the silica coated magnetic particles. The alkali metal salt also assists in the solubilization of the nucleic acids because of the kosmotropic effect that the salt has on the organization of the water molecules in solution around the nucleic acids. In some embodiments, such a complexing salt may be lithium chloride or lithium bromide. The salt may be present at a concentration of between 2-10 M (*e.g*., 2 M, 3 M, 4 M, 5 M, 6 M, 7 M, 8 M, 9 M or 10 M). In certain embodiments, lithium chloride is used in the Lysis solution at a concentration of 2 M (DNA Direct Lysis protocols). In certain embodiments, lithium chloride is used in the Lysis solution at a concentration of 8 M (DNA and RNA Lysate protocols). A Lysis solution additionally includes a detergent. Although any detergent (anionic, nonionic, cationic, and zwitterionic detergent) may be used, nucleic acid isolation is optimally achieved through the use of a nonionic detergent (DNA and RNA Lysate protocols) or an anionic detergent (DNA Direct Lysis protocols). Although any nonionic detergent may be used, examples of nonionic detergents are those from the Tween class (Tween-20, Tween-40, Tween-60, etc.), the Triton class (X-100, X-114, etc.), Tergitols, Nonidets or Igepal (NP-40, etc.). The nonionic detergent may be used at a concentration of 0.05-5 % (*e.g*., at about 0.1%, 0.3%, 0.5%, 1%). Although any anionic detergent may be used, and example of an anionic detergent is SDS (sodium dodecyl sulfate). The anionic detergent may be used at a concentration of 0.05-2 % (*e.g*., at about 0.1%, 0.3%, 0.5%, 1%).

A Lysis solution additionally includes a surfactant, such as diethylene glycol monoethyl ether (DGME). The surfactant may be used at a concentration of 5 - 40% (*e.g*., 10%, 15%, 20%, 30% or 40%). Through experimentation with the methods of the present invention, it was observed that in addition to helping solubilize all of the components into the Lysis solution, surfactants such as DGME alter the solvent properties of the Lysis solution by creating a less polar environment, such that binding of the nucleic acids to the silica coated magnetic particles is energetically favored.

A Lysis solution additionally includes a chelating agent to bind extraneous metal ions which are released during the lysing procedure. Metal ions may catalyze the degradation of nucleic acids under certain conditions. The chelating agent may be present at a concentration of 5 - 20 mM (*e.g*., 5 mM, 6 mM, 7 mK 8mM, 9 mM, 10 mM, 11 mM, 12 mM, 13 mM, 14 mM, 15 mM, 16 mM, 17 mM, 18 mM,19 mM or 20 mM). In some embodiments the chelating agent is EDTA (DNA and RNA Lysate protocols), and in other embodiments the chelating agent is citrate (DNA Direct Lysis protocol).

The Lysis solution additionally includes an antifoaming agent, such as a mixture of polydimethylsiloxane fluid and silica (*e.g*., Dow Coming^{®} Antifoam A compound).

The Lysis solution of the present invention is advantageous. The unique combination of a high concentration of a kosmotropic complexing salt and detergent in a neutral to high pH buffer inactivates enzymes harmful to nucleic acids (such as DNases and RNases), without the use of such reagents as phenol, chloroform, and guanidinium salts. A reducing agent, such as Tris (carboxyethyl) phosphine (TCEP) or beta-mercaptoethanol (BME) is also added to the Lysis Solution in the RNA Lysate protocols in order to further inactivate RNase enzymes. The preferred reducing agent is TCEP at concentrations at about 5-50 mM. Additionally, the Lysis solution confers a high nucleic acid binding property to silica coated magnetic particles through the use of high concentrations of a kosmotropic salt and the solvent polarity modifier DGME. The antifoaming agent, Dow Corning^{®} Antifoam A compound, allows for detergent action in the Lysis solution, while minimizing the foam produced while the lysate is pipetted in the instrument.

In one embodiment, the Lysis solution for the DNA Direct Lysis of blood cells is formulated to contain 50 mM Tris (pH 10-11), 2 M LiCl, 0.1% SDS, 30% DGME, 10 mM citrate and 0.05% Dow Corning^{®} Antifoam A compound.

In a second embodiment, the Lysis solution for the DNA Lysate protocols for cultured cells, white blood cell pellets, tissues, etc. is formulated to contain 100 mM Tris (pH 7), 8 M LiCl, 0.1% Triton X, 10% DOME, 10 mM EDTA and 0.01% Dow Corning^{®} Antifoam A compound.

In a third embodiment, the Lysis solution for the RNA Lysate protocols for cultured cells, white blood cell pellets, tissues, etc. is formulated to contain 100 mM Tris (pH 7), 8 M LiCl, 0.1 % Triton X, 10% DGME, 10 mM EDTA, 20 mM TCEP and 0.01% Dow Corning^{®} Antifoam A compound.

### Enzyme Solutions

In the DNA Direct Lysis protocols, the Magnetic Processing Tubes 18 contain Proteinase K. A suitable Proteinase K solution contains about 10-25 mg/mL Proteinase K (*e.g*., 10 mg/mL, 15 mg/mL, or 25 mg/mL). In one embodiment, a suitable Proteinase K solution contains 20 mg/mL of Proteinase K.

In the DNA Lysate protocols, RNase A enzyme is added to and incubated with the lysate prior to loading the lysate onto the instrument in order to eliminate RNA contamination from the DNA purification. A suitable RNase A solution contains about 2-20 mg/mL RNase A (*e.g*., 2 mg/mL, 10 mg/mL, 15 mg/mL, or 20 mg/mL). In one embodiment, a suitable RNase A solution contains 4 mg/mL of RNase A.

In the RNA Lysate protocols, DNase I enzyme is added to the silica coated magnetic particles after the first wash steps in order to eliminate DNA contamination from the RNA purification. A suitable DNase I enzyme solution contains 50 - 200 Units (*e.g*., 50 Units, 100 Units, 150 Units or 200 Units) of enzyme activity in 200 µL in a DNase Buffer. In one embodiment, a suitable DNase I solution contains 100 Units enzyme activity in 200 µL DNase Buffer.

### Binding Solutions

A Binding solution is used when purifying DNA from a sample using the DNA Direct Lysis method, since the Binding solution significantly increases the amount of kosmotropic salt, preferably a lithium salt, present in the lysate such that DNA binds to the silica coated magnetic particles to completion. After the blood cells are lysed in the magnetic processing tube in Lysis solution and Proteinase K, the binding solution is added to increase the salt concentration in the lysate in order to cause DNA binding. The present invention features a DNA Direct Lysis Binding solution that has the following components; a buffer, a kosmotropic salt, and a surfactant.

The first component of the Binding solution is a buffer that maintains the pH of the solution. For example, the pH may be at least about 7-10 (*e.g*., 7, 8, 9,10). The buffer may be used at a concentration of 50 -150 mM (*e.g*., 50 mM, 100 mM, or 150 mM). In some embodiments, Tris buffer is an appropriate buffer. For example, in some embodiments Tris may be used at a concentration of 0mM..

Another component of the Binding solution is a complexing salt that confers unique binding properties to nucleic acids (*e.g*., an RNA or DNA complexing salt), such that the nucleic acids preferentially bind to the silica coated magnetic particles, not only because the nucleic acids are charge neutralized by the lithium ions binding to the phosphate groups and therefore decreasing the solubility in solution, but also due in part to the kosmotropic effect the salt has on the organization of the water molecules in solution around the nucleic acids. In some embodiments, such a complexing salt may be a lithium salt, such as lithium chloride or lithium bromide. The salt may be present at a concentration of between 5 - 15 M (5 M, 6 M, 7 M, 8 M, 9 M,10 M, 11 M, 12 M, 13 M,14 M or 15 M) because preferential binding of DNA to a solid support is enhanced by high concentrations of complexing salts. For example, in some embodiments lithium chloride may be used at a concentration of 10 M.

Another component of the Binding solution is a surfactant In certain instances, DOME is used. The concentration may be 5 -15 % (*e.g*., 5%, 6%, 7%, 8%, 9%, 10%, 11%, 12%, 13%, 14% or 15%). For example, in some embodiments DGME may be used at a concentration of 10 %.

A Binding solution may optionally be used when purifying DNA or RNA using the DNA Lysate or RNA Lysate methods. The Binding solution may be similar in composition to the DNA Direct Lysis method such that it is composed of a lithium salt at a concentration of at least 5 M, a surfactant concentration of at least 5%, a buffer, and optionally an alcohol such as ethanol or isopropanol, or the Binding solution may be an alcohol such as ethanol or isopropanol.

A Rebinding solution is used in the RNA Lysate protocols after the DNase treatment. Optimum DNase activity requires the use of conditions that can cause the nucleic acids to dissociate from the solid support. A Rebinding solution is therefore added to the DNase solution containing the particles to ensure that RNA particles "re-bind" to the silica coated magnetic particles prior to removal of the combined DNase and Rebinding solutions. The Rebinding solution can be a combination of high salt, such as lithium chloride, at a concentration of about 5 to 15 M, or alcohol-containing solutions, or alcohol. In one embodiment the Rebinding solution is isopropyl alcohol.

### Wash Solutions

One or more Wash solutions may be used to wash the silica coated magnetic particles to which nucleic acids are bound, so as to rid them of non-nucleic acid contaminants such as proteins, phospholipids, heme, etc. The Wash solutions may contain an alcohol at a concentration greater than 50% (*e.g*., 55%, 60%, 70%, 80%, 90%, 95%, or 100%). The alcohol can be, for example, ethanol, methanol or isopropyl alcohol. In some embodiments, ethanol is used at a concentration of about 55% or 70%. In other embodiments, isopropyl alcohol is used at a concentration of about 100%. In further embodiments, methanol is used at a concentration of about 20%.

Wash I solution may be used in all protocols as the first wash solution after the lysate or lysate/binding solutions are pipetted off the particles, to substantially wash off contaminants such as blood components, cell membrane components, hemoglobin, heme, etc. The Wash I solution contains a high lithium salt concentration, such as lithium chloride, at a concentration between 4-10 M (*e.g*., 5-6 M, 4-7 M, 5-8 M, 6-9 M, 6-10 M, 7-10 M, 5 M, 6 M, 7 M, 8 M, 9 M, or 10M). A high salt concentration means a salt concentration high enough to inhibit RNase enzyme activity and cause the nucleic acid to remain bound to the magnetic particles. The Wash I solution additionally contains an alcohol (*e.g*. ethanol, methanol or isopropyl alcohol). The alcohol concentration is at 15-80 % (*e.g*., 15-20%, 20-30%, 30-40%, 40-50%, 3545%, 55-65%. 60-70%, 65-75%, 70-80%, 15%, 18%, 20%, 30%, 40%, 55%, 60%, 70%, or 75%). Wash I solution may contain ethanol at a concentration of 55%. Wash I solution may also contain 5 M LiCl and 55% ethanol.

A Nash solution II may be used. If so, Wash II solution is used only in the DNA Direct Lysis Protocols to wash away less polar compounds such as lipids from the silica coated magnetic particles. Due to the large amount of cellular material in the Direct Lysis methods there is a substantial amount of lipid-like substances which contaminate the particles after the Wash I step(s) are completed. The Wash II solution contains an alcohol. In certain embodiments the Wash II solution is 100% isopropyl alcohol.

The Wash III solution may be used in all protocols as a "desalting wash" and further removes residual biological-material prior to the DNase step in the RNA protocols or prior to the elution steps in all protocols. The Wash III solution contains a buffer, alcohol, and a chelator (*e.g*. EDTA, CDTA, or citrate). The buffer composition may be Tris, at about pH 6 - 9 (*e.g*., pH 6.5, 7, 7.5, 8.0 or 8.5). The buffer may be at a concentration of 50-150 mM (*e.g*., 60 mM, 70 mM, 80 mM, 90 mM, 100 mM, 120 mM, or 140 mM). Wash III solution additionally contains an alcohol at 50 - 90 % (*e.g*., 55-65%, 60-70%, 65-75%, 70-80%, 55%, 60%, 60%, 75%, 80%, or 85%). The chelator concentration may be at 1- 20 mM. Wash III solution may contain 70% ethanol,100 mM Tris (pH 6-8) and 5 mM EDTA.

The Wash IV solution may be used in the RNA Lysate protocols to eliminate any final RNase enzyme activity which may be remaining after the DNase step has been executed. It is an RNase denaturing wash which contains a high lithium salt concentration, preferably lithium chloride, at a concentration between 4 and10 M. For the purposes of the present invention, a high salt concentration means a salt concentration high enough to inhibit RNase enzyme activity and cause the nucleic acid to remain bound to the magnetic particles. The Wash IV solution additionally contains an alcohol (*e.g*., ethanol, methanol or isopropyl alcohol). The alcohol concentration is at 5 - 30 %. Wash IV solution may contain methanol at a concentration of 18%. Wash IV solution may contain 6 M LiCl and 18% methanol.

### Elution Solutions

Substantially undegraded nucleic acids (*e.g*., DNA or RNA) that are bound to the solid support as a result of the isolation procedure can be eluted using an Elution Solution (also called a Hydration Solution). The simplicity of the reagents used in lysing the biological material and binding of the nucleic acid to the solid support, by the present invention as well as in in washing the solid support taught lends itself to a simple Elution Solution. A variety of Elution Solutions are known to those having ordinary skilled in the art. In some embodiments, Versagene™ DNA Elution Solution (Gentra Systems, Inc., Minneapolis, MN) may be used for eluting bound substantially undegraded DNA. In some instances, Tris-EDTA (TE) may be used for eluting bound substantially undegraded DNA.

Substantially undegraded RNA, which is bound to the solid support, may be eluted using an RNA Elution Solution. In some instances, Versagene™ RNA Elution Solution (Gentra Systems, Inc., Minneapolis, MN) may be used for eluting bound substantially undegraded RNA. RNase-free water may be used to elute bound substantially undegraded RNA. Water may be treated with a substance that inactivates RNases, such as diethyl pyrocarbonate (DEPC), and used for eluting RNA. Other RNA Elution Solutions known to those having ordinary skill in the art also may be used.

### Purification/Isolation Methods.

The present invention also provides methods for purifying DNA and RNA from material (*e.g*., biological material).

In some instances, the Lysis Solution is used to lyse the material (*e.g*., biological material) and release nucleic acids into a lysate, before adding the lysate to the solid support (*e.g*., magnetic particles contained in Magnetic Processing Tubes 18). Additionally, the Lysis Solution prevents the deleterious effects of harmful enzymes such as nucleases. In some instances, the Lysis Solution is mixed with the material and the solid support (*e.g*., magnetic particles contained in Magnetic Processing Tubes 18). Enzymes such as RNase, DNase, or Proteinase K may be added following lysis, or alternatively, they may be added directly to tube with the solid support or added to the Lysis Solution to degrade contaminating RNA, DNA or proteins present in the sample. In some embodiments of purifying DNA, following lysis, the binding of the nucleic acid to the solid support can be improved by employing a Binding Solution.

In the DNA Direct Lysis protocol, the sample is lysed on the instrument and Binding solution is added to the lysate. Following lysis of the sample (*e.g*., blood, buffy coat, etc) and binding of the DNA, any remaining biological materials (proteins, phospholipids, *etc.*) are removed by applying a magnetic field to the magnetic particles to draw the particle bound DNA away from the remaining lysate. The lysate is aspirated away from the particles and deposited in the waste tube. Wash I solution is added to the particle pellet one or more times, and each time the particles are mixed into the wash by pipetting. The particles are then drawn to the magnet and the Wash I solution is aspirated away from the particles. The same procedure is followed for the Wash II and Wash III solutions, such that any remaining biological contaminants and finally salts from the high salt containing wash solutions are washed from the particles prior to elution. Subsequently, bound DNA is eluted using an adequate amount of an Elution solution known to those having ordinary skill in the art. The particles are pipetted in the Elution solution to mix and the eluate containing the DNA is collected.

In the DNA Lysate protocol, the sample is lysed prior to loading it onto the instrument and treated with RNase enzyme. Following lysis of the sample (*e.g*., cultured cells, white blood cells, tissue) the lysate is loaded onto the instrument, a Binding solution is optionally added to the lysate, and DNA is bound to the particles. Any remaining biological materials (proteins, phospholipids, *etc.)* are removed by applying a magnetic field to the magnetic particles to draw the particle bound DNA away from the remaining lysate. The lysate is aspirated away from the particles and deposited in the waste tube. Wash I solution is added to the particle pellet one or more times, and each time the particles are mixed into the wash by pipetting. The particles are then drawn to the magnet and the Wash I solution is aspirated away from the particles. The same procedure is followed for the Wash III solution, such that any remaining biological contaminants, and salts from the high salt containing Wash I solution, are washed from the particles prior to elution. Subsequently, bound DNA is eluted using an adequate amount of an Elution solution known to those having ordinary skill in the art. The particles are pipetted in the Elution solution to mix and the eluate containing the DNA is collected.

In the RNA Lysate protocol, the sample is lysed prior to loading it onto the instrument, and following lysis of the sample (*e.g*., cultured cells, white blood cells, tissue) the lysate is loaded onto the instrument, a Binding solution is optionally added to the lysate, and nucleic acids (total nucleic acids) are bound to the particles. Any remaining biological materials (proteins, phospholipids, *etc.*) are removed by applying a magnetic field to the magnetic particles to draw the particle bound nucleic acids away from the remaining lysate. The lysate is aspirated away from the particles and deposited in the waste tube. Wash I solution is added to the particle pellet one or more times, and each time the particles are mixed into the wash by pipetting. The particles are then drawn to the magnet and the Wash I solution is aspirated away from the particles. The same procedure is followed for the Wash III solution, such that any remaining biological contaminants, and salts from the high salt containing Wash I solution, are washed from the particles prior to the DNase step. A buffered DNase solution is added and mixed into the particle pellet and incubated, after which a Rebinding solution is also added to the DNase and particle mixture. The particles are drawn to the magnet and the solution mixture is aspirated from the particles. Subsequently, Wash IV and Wash III are added one or more times, mixed and aspirated away from the particles. The Wash IV removes any remaining RNase enzyme activity from the particles and Wash III removes any remaining biological contaminants, and salts from the high salt containing Wash IV solution. The bound RNA is eluted using an adequate amount of an Elution solution known to those having ordinary skill in the art. The particles are pipetted in the Elution solution to mix and the eluate containing the RNA is collected.

### Methods of Isolating Nucleic Acids Using the Automated Instrument.

In certain embodiments, samples such as blood, cultured cells, body fluids, etc. are manually transferred from the primary collect tube into Magnetic Processing Tube 18 by the operator. The Magnetic Processing Tubes 18 contain magnetic particles and may optionally contain proteinase K. Magnetic Processing Tubes 18 containing samples and particles (and optionally enzymes) are placed in the Magnetic Processing Holder 16 located on the mounting plate of the Process Assembly 10 in the most rearward position (far back row). One or more samples can be loaded. Magnetic Processing Tubes1 8, Disposable Tips 26, Waste Tubes 22, and Output Tubes 20 are loaded into corresponding sections of the Mounting Plate 12 of the Process Assembly 10. Pre-packaged Reagent Packs 24 appropriate for desired protocol are loaded into the instrument. The desired protocol is selected through the User Interface 82. The system provides the capability to of processing multiple user-selectable protocols. Each protocol provides the process parameters that have been optimized for the desired sample type and volume.

In one embodiment, the sample volume for the Direct Lysis of whole blood or buffy coat may be 200 µL up to 800 µL, for example, 200 µL or 500 µL, up to 800 µL. In one embodiment, the sample volume for the isolation of nucleic acids from cultured cell lysates may be 1,000 cells up to 10 million cells, for example 1,000 cells up to 5 million cells, or from 1 million to 2 million cells. In another embodiment, the sample volume for the DNA or RNA Lysate protocol for whole blood may be 500 µL to 3 mL.

Once the sample (or samples) is transferred from the primary sample tube to the Magnetic Process Tubes 18, the operator installs the remaining disposables and Reagent Pack 24 onto the Mounting Plate 12 of the Process Assembly 10. The operator activates the automated instrument, allowing the instrument to initiate the purification process so that it goes through the required sequential steps.

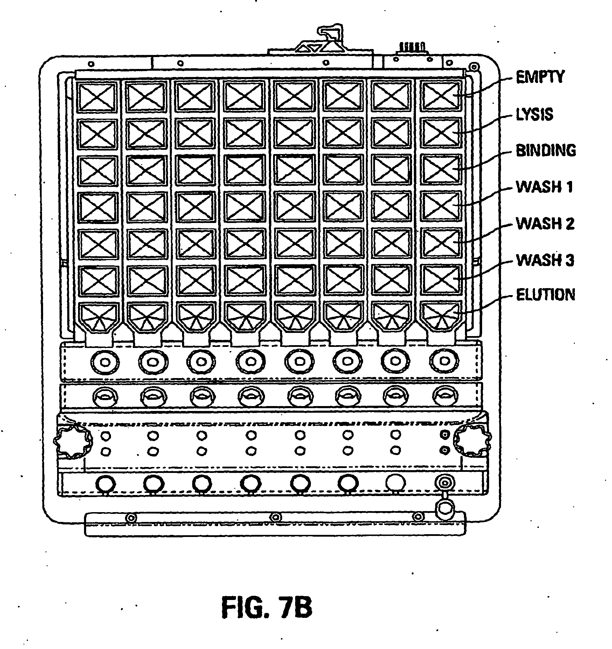

The following protocol is used for processing DNA from 200 µL or 500 µL samples of whole blood or buffy coat using the Direct Lysis protocol. First, the operator adds 200 µL or 500 µL of whole blood or buffy coat to a 10 mL Magnetic Processing Tube 18 containing Proteinase K and magnetic particles in glycerol. The operator loads all disposables (tips, tubes) and either a "DNA Whole Blood" or a "DNA Buffy Coat" Reagent Pack 24 into the holders along the Mounting Plate 12 of the Process Assembly 10 of the instrument. The disposable loading order, front to back of instrument, is set forth as depicted in Figure 7B, namely: Output tube 20, Disposable Tips 26, Magnetic Processing Tube 18, Waste Tube 22, and Reagent Pack(s) 24.

The instrument performs the following steps: loads Disposable Tips 26 onto Aspiration Pipettor Assembly 38; verifies the presence, number and volume of sample; using the Dispense Pipettor Assembly 48, adds Lysis Solution to sample(s) in the Magnetic Processing Tubes 18; using Aspiration Pipettor Assembly 38, pipettes up and down to mix; incubates samples at 45°C for ten minutes (during incubation, the sample is mixed); using Aspiration Pipettor Assembly 38, transfers waste to Waste Tube 22; using Dispense Pipettor Assembly 48, adds Binding Solution to sample and using Aspiration Pipettor Assembly 38, pipettes up and down 10 times to mix; incubates at room temperature for 10 minutes; Magnetic Elevator Assembly 28 brings the Magnet 32 into position against the Magnetic Processing Tube 18, incubates for 20 seconds, and transfers waste to Waste Tube 22 using Aspiration Pipettor Assembly 38; using Dispense Pipettor Assembly 48, adds 500 µL to 1 mL Wash I Solution to sample; Magnetic Elevator Assembly 28 brings Magnet 32 into position against the Magnetic Processing Tube 18, incubates for 20 seconds, and transfers waste to Waste Tube 22 using Aspiration Pipettor Assembly 38 (previous two steps may be repeated, if desired); using Dispense Pipettor Assembly 48, adds 500 µL to 1 mL Wash II Solution to sample; Magnetic Elevator Assembly 28 brings Magnet 32 into position against the Magnetic Processing Tube 18, incubates for 20 seconds, and transfers waste to Waste Tube 22 using Aspiration Pipettor Assembly 38 (previous two steps may be repeated, if desired); Wash III is handled similarly to the descriptions for Wash I and Wash II; using Dispense Pipettor; adds 100 µL Elution Solution to sample and using Aspiration Pipettor Assembly 38, pipettes up and down multiple times to mix; incubates at room temperature for 5 minutes; and Magnetic Elevator Assembly 28 brings Magnet 32 into position against the Magnetic Processing Tube 18, incubates for 1 minute, and transfers DNA to Output Tube 20 using Aspiration Pipettor Assembly 38(elution step may be repeated, if desired to increase DNA yield).

### Articles of Manufacture

The reagents and methods featured in the present invention provide substantially pure and undegraded nucleic acids with relatively little contaminating impurities such that the nucleic acids may be used in downstream processes known to those having ordinary skill in the art. Substantially pure, undegraded nucleic acids are nucleic acids that is suitable for use in subsequent analyses, including, but not limited to, nucleic acid quantification, restriction enzyme digestion, DNA sequencing, hybridization technologies, such as Southern Blotting, *etc.*, amplification methods such as Polymerase Chain Reaction (PCR), Ligase Chain Reaction (LCR), Nucleic Acid Sequence Based Amplification (NASBA), Self-sustained Sequence Replication (SSR or 3SR), Strand Displacement Amplification (SDA), and Transcription Mediated Amplification (TMA), Quantitative PCR (qPCR), or other DNA analyses, as well as RT-PCR, in vitro translation, Northern blotting, microarray analysis and other RNA analyses.

This invention will be further described by reference to the detailed examples included herein. These examples are offered to further illustrate the various specific and illustrative embodiments and techniques.

### EXAMPLES

### Example 1- Preparation of Magnetic Processing Tubes

The tube manufacturing process for the 200 µL DNA Direct Lysis Whole Blood protocol process tubes consisted of 7 µL (20 mg/mL) of Proteinase K solution dispensed into the bottom of a 10 mL conical tube, followed by 46 µL Promega Magnesil^{®} paramagnetic particle solution and 11 µL of glycerol (Sigma). The tubes were dried in a vacuum oven at 28°C for three to five hours to form a Magnetic Processing Tube containing 4.6 mg of particles suspended in about 100% glycerol and Proteinase K.

The tube manufacturing process for the 200 µL and 500 µL DNA Direct Lysis Buffy protocols, and the 500 µL DNA Direct Lysis Whole Blood protocol process tubes consisted of 15 µL (20 mg/mL) of Proteinase K solution dispensed into the bottom of a 10 mL conical tube, followed by 130 µL Promega Magnesil^{®} paramagnetic particle solution and 28 µL of glycerol (Sigma). The tubes were dried in a vacuum oven at 30°C eight to sixteen hours to form a Magnetic Processing Tube containing 13 mg of particles suspended in about 100% glycerol and Proteinase K.

The tube manufacturing process for the RNA Lysate and DNA Lysate protocols consisted of 130 µL Promega Magnesil^{®} paramagnetic particle solution and 33 µL of glycerol (Sigma) dispensed into the bottom of a 10 mL conical tube. The tubes were dried in a vacuum oven at 30°C eight to sixteen hours to form a Magnetic Processing Tube containing 13 mg of particles suspended in about 100% glycerol.

### Example 2 - Isolation and Purification of RNA from Cultured Cells,

### White Blood Cells or Tissue Using the RNA Lysate Protocol

In this example, RNA from 1 million and 5 million eukaryotic K562 cell pellets were purified using the RNA Lysate Protocol. White blood cells or tissue can also be purified using this protocol. The use of Binding solution was examined in order to determine whether a yield improvement was observed for these cell numbers when a Binding solution was added to the lysate after homogenization. This is an automated instrument protocol, but one or more of these steps can also be executed manually.

*Lysate Generation.* Cell pellets containing 1 million and 5 million eukaryotic K562 cells were lysed in 400 µL of Lysis Solution (8 M LiCI, 0.1% Triton X-100,10% DGME, 10 mM EDTA, 0.01 % Dow Corning^{®} Antifoam A compound, 20 mM TCEP in 100 mM Tris at pH 7.0). The cells were vortexed for 1 minute to lyse and homogenize the samples.

*RNA Binding to Particles.* Each lysate was transferred to a Magnetic Processing Tube containing particles and loaded onto the instrument. The samples were mixed into the particles by pipetting 30 times. To test the effect of using a Binding Solution to improve RNA yields, Binding Solution was added to the particle and lysate mixture in half the tubes (for 1 million cells, 600 µL 100 % ethanol; for 5 million cells, 200 µL of 55% ethanol, 5 M LiCl) and mixed into the mixture by pipetting 30 times, while no Binding Solution was added to the rest of the samples. The magnet was moved into position against the tube, and particles removed from the solution to the side of the tube. Waste was aspirated away from the particles into the waste tube and the magnet was removed from the tube.

*First Wash.* 800 µL of Wash I Solution (55% ethanol, 5 M LiCl) was added to the particles and mixed by pipetting 10 times. The magnet was moved into position against the tube, and particles removed from the solution to the side of the tube. Waste was aspirated away from the particles into the waste tube and the magnet was removed from the tube.

*Second Wash.* 500 µL of Wash III Solution (70% ethanol, 100 mM Tris, 5 mM EDTA, pH 7) was added to the particles and mixed by pipetting 10 times. The magnet was moved into position against the tube, and particles removed from the solution to the side of the tube. Waste was aspirated away from the particles into the waste tube and the magnet was removed from the tube.

*DNase Treatment.* 200 µL of DNase Solution (100 Units/200 µL in DNase buffer: 6 mM CaCl₂, 15 mM MgCl₂, 20 mM Tris) was added to the particles, mixed by pipetting 20 times and incubated at room temperature for 20 minutes. The solution was mixed twice during this incubation period.

*RNA Rebinding to Particles.* 200 µL of Rebinding Solution (100% Isopropanol) was added to the DNase and particle mixture, and mixed by pipetting 20 times. The magnet was moved into position against the tube, and particles removed from the solution to the side of the tube. Waste was aspirated away from the particles into the waste tube and the magnet was removed from the tube.

*Third Wash.* 800 µL of Wash IV Solution (18% methanol, 6 M LiCl) was added to the particles and mixed by pipetting 10 times. The magnet was moved into position against the tube, and particles removed from the solution to the side of the tube. Waste was aspirated away from the particles into the waste tube and the magnet was removed from the tube. This wash was repeated a second time.

*Final Wash* 500 µL of Wash III Solution (70% ethanol, 100 mM Tris, 5 mM EDTA, pH7) was added to the particles and mixed by pipetting 10 times. The magnet was moved into position against the tube, and particles removed from the solution to the side of the tube. Waste was aspirated away from the particles into the waste tube and the magnet was removed from the tube. This wash was repeated a second time.

*Sample Elution.* 100 µL of Tris-EDTA Solution (10 mM Tris, pH 7.5, 0.1 mM EDTA) was added to the particles and mixed by pipetting 40 times. The sample was incubated for 5 minutes. The magnet was moved into position against the tube, and particles removed from the solution to the side of the tube. The solution containing RNA was aspirated and transferred to an output tube. The elution step was repeated a second time and the two elution volumes were combined into one final output tube.

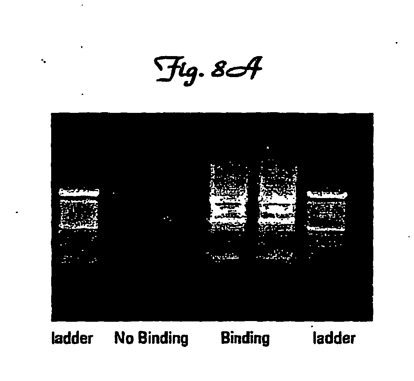

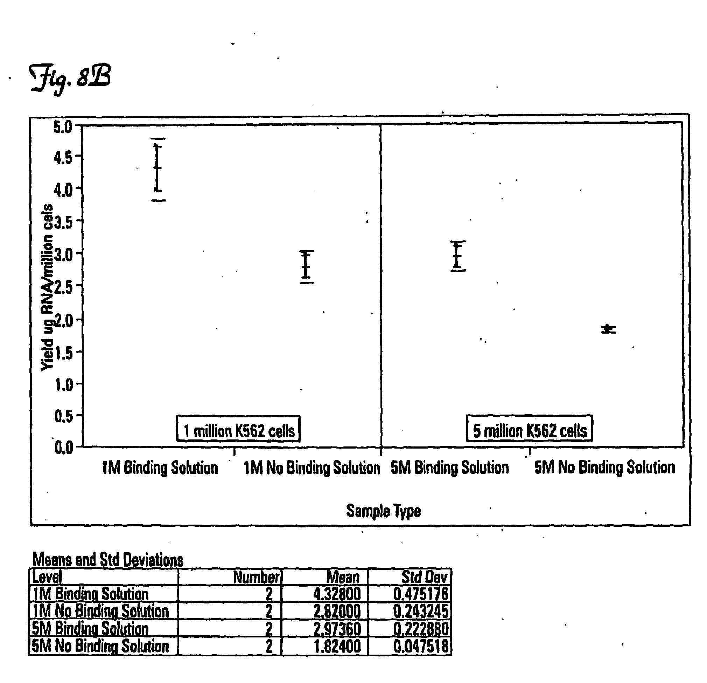

*Results.* The purified RNA samples were analyzed by agarose gel electrophoresis for stable and intact ribosomal RNA bands (Figure 8A). 15 µL of each 5 million pellet sample was loaded onto a 1.4% agarose gel and electrophoresed at 80 V for 45 minutes. The results indicated that clean (*i.e*., intact and essentially free of DNA) and stable RNA was obtained from cultured cells. Graph 1 contains the yield data from 1 million and 5 million K562 cell pellets in µg RNA per million cells as determined by UV absorbance measurements at 260 nm.

The data in Graph 1 indicates that higher yields of RNA are obtained by adding a Binding Solution to the lysates prior to lysate aspiration and the subsequent washing steps. The gel for 5 million cultured cells shows the difference in ribosomal band intensity with and without the Binding solution. This experimental work showed that the methods and solutions of the present invention provide fast and effective methods for purifying RNA.

### Example 3: Isolation and Purification of RNA from Cultured Cells or Tissue using RNA Lysate Protocol

This example contains a protocol for the purification of RNA from Cultured Cells or tissue. Some cell lines or tissue may require TCEP as a reducing agent in the Lysis solution in order to more thoroughly eliminate RNase enzyme activity.
This is an automated instrument protocol, but one or more of these steps can also be executed manually.

*RNA Binding to Particles.* The cells in the sample were lysed using 800 µL Lysis Solution (8 M LiCl, 0.1% Triton X-100, 10% DGME,10 mM EDTA, in 100 mM Tris at pH 7.0). The lysate was transferred to a Magnetic Processing Tube containing particles (100 µL Promega Magnesil^{®}paramagnetic particles) and loaded onto the instrument. The lysate and particles were mixed together by pipetting 20 times. The magnet was moved into position against the tube, and particles removed from the solution to the side of the tube. Waste was aspirated away from the particles into the waste tube and the magnet was removed from the tube.

*First Wash.* 800 µL of Wash I solution (55% ethanol, 5 M LiCl) was added to the particles and mixed by pipetting 10 times. The magnet was moved into position against the tube, and particles removed from the solution to the side of the tube. Waste was aspirated away from the particles into the waste tube and the magnet was removed from the tube.

*Second Wash.* 500 µL of Wash III Solution (70% ethanol, 100 mM Tris, 5 mM EDTA, pH 7) was added to the particles and mixed by pipetting 10 times. The magnet was moved into position against the tube, and particles removed from the solution to the side of the tube. Waste was aspirated away from the particles into the waste tube and the magnet was removed from the tube.

*DNase Treatment.* 200 µL of DNase Solution (100 Units/200 µL in DNase buffer: 6 mM CaCl₂,15 mM MgCl₂, 20 mM Tris) was added to the particles, mixed by pipetting 20 times and incubated at room temperature for 10 minutes. The solution was mixed twice during this incubation period.

*RNA Rebinding to Particles.* 200 µL of Rebinding Solution (100% Isopropanol) was added to the DNase and particle mixture, and mixed by pipetting 20 times. The magnet was moved into position against the tube, and particles removed from the solution to the side of the tube. Waste was aspirated away from the particles into the waste tube and the magnet was removed from the tube.

*Final Wash.* 800 µL of Wash III Solution (70% ethanol, 100 mM Tris, 5 mM EDTA, pH 7) was added to the particles and mixed by pipetting 10 times. The magnet was moved into position against the tube, and particles removed from the solution to the side of the tube. Waste was aspirated away from the particles into the waste tube and the magnet was removed from the tube. This wash was repeated a second time.

*Sample Elution.* 100 µL of Tris-EDTA Solution (10 mM Tris, pH 7.5, 0.1 mM EDTA) was added to the particles and mixed by pipetting 40 times. The sample was incubated for 5 minutes. The magnet was moved into position against the tube, and particles removed from the solution to the side of the tube. The solution containing RNA was aspirated and transferred to an output tube. The elution step was repeated a second time and the two elution volumes were combined into one final output tube.

### Example 4 - Isolation and Purification of DNA from Cultured Cells,

### White Blood Cells or Tissue Using the DNA Lysate Protocol

In this example, DNA from 5 million eukaryotic K562 cell pellets were purified using the DNA Lysate Protocol. White blood cells or tissue can also be purified using this protocol. The use of RNase A enzyme to remove unwanted RNA contamination was examined by gel electrophoresis and compared to samples in which RNase A enzyme was not used in the purification. In this example, a Binding Solution was not added to the lysate, however adding a Binding Solution to the lysate increases DNA yields similarly to the increase in RNA yields observed in Example 2. This is an automated instrument protocol, but one or more of these steps can also be executed manually.

### Lysate Generation.

Cell pellets containing 5 million eukaryotic K562 cells were lysed in 600 µL of Lysis Solution (8 M LiCl, 0.1% Triton X-100, 10% DGME, 10 mM EDTA, 0.01% Dow Corning^{®} Antifoam A compound, in 100 mM Tris at pH 7.0). The cells were vortexed for 1 minute to lyse and homogenize the samples. The Lysate was treated with 2 µL of 4 mg/mL RNase A solution and incubated for 10 minutes.

*DNA Binding to Particles.* Each lysate was transferred to a Magnetic Processing Tube containing particles and loaded onto the instrument. The samples were mixed into the particles by pipetting 20 times. The magnet was moved into position against the tube, and particles removed from the solution to the side of the tube. Waste was aspirated away from the particles into the waste tube and the magnet was removed from the tube.

*First Wash.* 800 µL of Wash I Solution (55% ethanol, 5 M LiCl) was added to the particles and mixed by pipetting 10 times. The magnet was moved into position against the tube, and particles removed from the solution to the side of the tube. Waste was aspirated away from the particles into the waste tube and the magnet was removed from the tube. This wash step is repeated at least once.

*Second Wash.* 800 µL of Wash III Solution (70% ethanol, 100 mM Tris, 5 mM EDTA, pH 7) was added to the particles and mixed by pipetting 10 times. The magnet was moved into position against the tube, and particles removed from the solution to the side of the tube. Waste was aspirated away from the particles into the waste tube and the magnet was removed from the tube. This wash step is repeated at least once.

*Sample Elution.* 200 µL of Tris-EDTA Solution (10 mM Tris, pH 7.5, 0.1 mM EDTA) was added to the particles and mixed by pipetting 40 times. The sample was incubated for 5 minutes. The magnet was moved into position against the tube, and particles removed from the solution to the side of the tube. The solution containing DNA was aspirated and transferred to an output tube. The elution step was repeated a second time and the two elution volumes were combined into one final output tube.

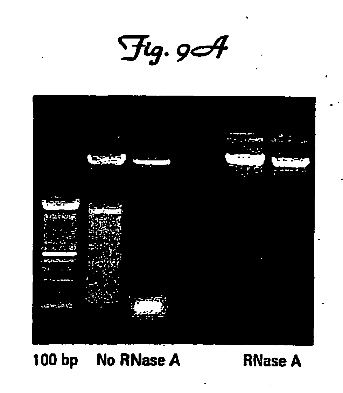

*Results.* The purified samples were analyzed by agarose gel electrophoresis for genomic DNA and to examine for RNA contamination in samples treated and untreated with RNase A (Figure 9A). 20 µL of each sample was loaded onto a 1.4% agarose gel and electrophoresed at 100 V for 60 minutes.

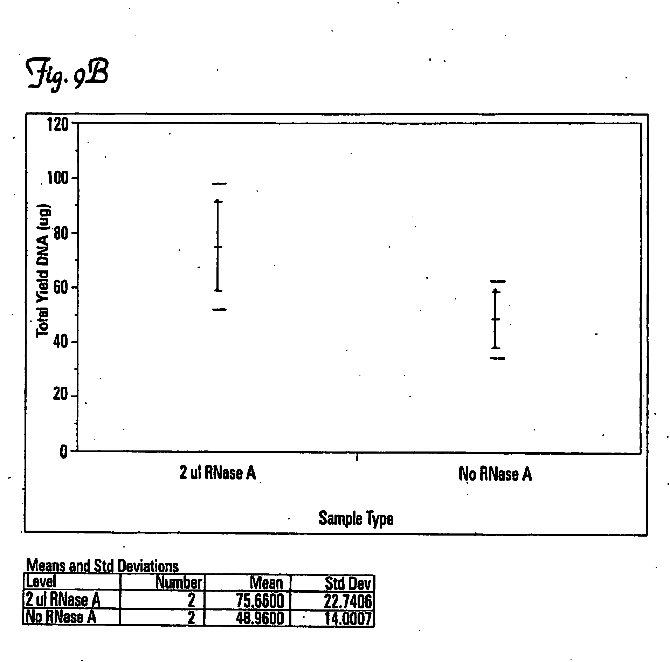

Both Figure 9B and the gel electrophoresis results (Figure 9A) both indicate that higher yields of genomic DNA are obtained by adding RNase A to the lysate to eliminate RNA contamination from the lysate, prior to binding to the particles, even though the non-treated RNase A group has UV absorbance contribution from RNA in the final yield calculation. It is apparent that if RNA is not removed from the lysate prior to DNA binding that the RNA competes for DNA binding sites which would otherwise be available. This experimental work showed that the methods and solutions of the present invention provide fast and effective methods for purifying DNA.

### Example 5 - Isolation and Purification of DNA from Cultured Cells

### Using DNA Lysate Protocol

In this example cultured cells or tissue can be purified using the DNA Lysate protocol. This is an automated instrument protocol, but one or more of these steps can also be executed manually.

*Lysate Generation.* Cells were lysed in 800 µL of Lysis Solution (8 M LiCl, 0.1% Triton X-100, 10% DGME, 10 mM EDTA in 100 mM Tris at pH 7.0). The samples were homogenized for 1 minute. The Lysate was treated with 2 µL of 4 mg/mL RNase A solution and incubated for 10 minutes.

*DNA Binding to Particles.* Each lysate was transferred to a Magnetic Processing Tube containing 90 µL of particles (silica coated ferrous oxide particles) and loaded onto the instrument. The samples were mixed into the particles by pipetting 20 times. The magnet was moved into position against the tube, and particles removed from the solution to the side of the tube. Waste was aspirated away from the particles into the waste tube and the magnet was removed from the tube.

*Wash.* 800 µL of Wash III Solution (70% ethanol, 100 mM Tris, 5 mM EDTA, pH 7) was added to the particles and mixed by pipetting 10 times. The magnet was moved into position against the tube, and particles removed from the solution to the side of the tube. Waste was aspirated away from the particles into the waste tube and the magnet was removed from the tube.

*Sample Elution.* 200 µL of Tris-EDTA Solution (10 mM Tris, pH 7.5, 0.1 mM EDTA) was added to the particles and mixed by pipetting 40 times. The sample was incubated for 5 minutes. The magnet was moved into position against the tube, and particles removed from the solution to the side of the tube. The solution containing DNA was aspirated and transferred to an output tube. The elution step was repeated a second time and the two elution volumes were combined into one final output tube.

### Example 6 - Isolation and Purification of DNA from Whole Blood using the Direct Lysis Protocol

In this example, DNA from 200 µL of whole blood was purified on the instrument using the Direct Lysis Protocol. DNA from 500 µL of whole blood can be obtained using a similar protocol, using increased Lysis and Binding Solution volumes and extra wash steps. This is an automated instrument protocol, but one or more of these steps can also be executed manually.

*Sample Lysis and Binding Steps.* A 200 µL blood sample was pipetted into a Magnetic Processing Tube containing particles and Proteinase K in glycerol and loaded onto the instrument. 600 µL of Lysis Solution (50 mM Tris, pH 10-11,2 M LiCl, 0.1% SDS, 30% DGME, 10 mM citrate, 0.05% Dow Corning^{®} Antifoam A compound) was added to the particles and the sample was mixed by pipetting 20 times. The sample was incubated at 45°C for 10 minutes with periodic mixing during the incubation. The lysed whole blood sample was moved to a second processing tube and 1200 µL of Binding Solution (50 mM Tris, 10 M LiCl, 10% DGME) was added to the lysate and mixed by pipetting 20 times. The magnet was moved into position against the tube, and particles removed from the solution to the side of the tube. Waste was aspirated away from the particles into the waste tube and the magnet was removed from the tube.

*First Wash.* 500 µL of Wash I Solution (55% ethanol, 5 M LiCl) was added to the particles and mixed by pipetting 15 times. The magnet was moved into position against the tube, and particles removed from the solution to the side of the tube. Waste was aspirated away from the particles into the waste tube and the magnet was removed from the tube. This wash was repeated a second time.

*Second Wash.* 800 µL of Wash II Solution (100% isopropanol) was added to the particles and mixed by pipetting 10 times. The magnet was moved into position against the tube, and particles removed from the solution to the side of the tube. Waste was aspirated away from the particles into the waste tube and the magnet was removed from the tube.

*Third Wash.* 500 µL of Wash III Solution (70% ethanol, 100 mM Tris, 5 mM EDTA, pH7) was added to the particles and mixed by pipetting 10 times. The magnet was moved into position against the tube, and particles removed from the solution to the side of the tube. Waste was aspirated away from the particles into the waste tube and the magnet was removed from the tube. This wash was repeated a second time.

*Elution Prewash (Ethanol Removal Step).* 200 µL of Tris-EDTA Solution (10 mM Tris, pH 7.5, 0.1 mM EDTA) was washed over the particle pellet while the magnet was next to tube and quickly aspirated and transferred to the waste tube.

*Sample Elution.* 100 µL of Tris-EDTA Solution (10 mM Tris, pH 7.5, 0.1 mM EDTA) was added to the particles and mixed by pipetting 150 times while the sample was incubated for 5 minutes. The magnet was moved into position against the tube, and particles removed from the solution to the side of the tube. The solution containing DNA was aspirated and transferred to an output tube: The elution step was repeated a second time and the two elution volumes were combined into one final output tube.

*Results.* Two instrument runs of 8 purified samples each were analyzed by agarose gel electrophoresis for genomic DNA (Gel 3). 15 µL of each sample was loaded onto a 1 % agarose gel and electrophoresed at 100 V for 60 minutes.

The data in Graph 3 shows DNA yields in µg for two instrument runs of 8 samples each. The results indicate that the automated methods provided consistent and competitive yields of clean and stable DNA from whole blood, showing that the compact instrument of the present invention provides fast and effective methods for purifying DNA.

### Example 7 - Isolation and Purification of DNA from Buffy Coat using the Direct Lysis Protocol

In this example, white blood cells were concentrated from whole blood by centrifugation and the layer of concentrated white blood cells were pulled from the tube for DNA purification by the Direct Lysis method. The protocols for 200 µL and 500 µL of buffy coat are similar to those of Direct Lysis of whole blood, but with increased Lysis and Binding Solution volumes and extra wash steps. It was necessary to increase Lysis and Binding Solution volumes as white blood cell numbers increased, in order to get higher DNA yields. Four samples of 500 µL buffy coat samples were processed, two using 2000 µL of Lysis Solution and 3200 µL of Binding Solution, and two using 1500 µL of Lysis Solution and 3000 µL of Binding Solution, in order to determine which set of conditions was higher yielding for DNA. This is an automated instrument protocol, but one or more of these steps can also be executed manually.

*Sample Lysis and Binding Steps.* A 500 µL buffy coat sample was pipetted into a Magnetic Processing Tube containing magnetic particles and Proteinase K in glycerol and loaded onto the instrument. 2000 µL or 1500 µL of Lysis Solution (50 mM Tris, pH 10-11, 2 M LiCl, 0.1% SDS, 30% DGME, 10 mM citrate, 0.05% Dow Coming^{®} Antifoam A compound) was added to the particles and mixed by pipetting 20 times. The sample was incubated at 45°C for 10 minutes with periodic mixing during the incubation. The lysed whole blood sample was moved to a second processing tube and 3200 µL or 3000 µL of Binding Solution (50 mM Tris, 10 M LiCl, 10% DGME) was added to the lysate and mixed by pipetting 30 times. The magnet was moved into position against the tube, and particles removed from the solution to the side of the tube. Waste was aspirated away from the particles into the waste tube and the magnet was removed from the tube.

*First Wash.* 1000 µL of Wash I Solution (55% ethanol, 5 M LiCl) was added to the particles and mixed by pipetting 15 times. The magnet was moved into position against the tube, and particles removed from the solution to the side of the tube. Waste was aspirated away from the particles into the waste tube and the magnet was removed from the tube. This wash was repeated a second time with 500 µL of Wash I Solution.

*Second Wash.* 800 µL of Wash II Solution (100% isopropanol) was added to the particles and mixed by pipetting 10 times. The magnet was moved into position against the tube, and particles removed from the solution to the side of the tube. Waste was aspirated away from the particles into the waste tube and the magnet was removed from the tube.

*Third Wash.* 1000 µL of Wash III Solution (70% ethanol, 100 mM Tris, 5 mM EDTA, pH 7) was added to the particles and mixed by pipetting 10 times. The magnet was moved into position against the tube, and particles removed from the solution to the side of the tube. Waste was aspirated away from the particles into the waste tube and the magnet was removed from the tube. This wash was repeated a second time with 500 µL of Wash III Solution.

*Elution Prewash (Ethanol Removal Step).* 200 µL of Tris-EDTA Solution (10 mM Tris, pH 7.5, 0.1 mM EDTA) was washed over the particle pellet while the magnet was next to tube and quickly aspirated and transferred to the waste tube.

*Sample Elution.* 400 µL of Tris-EDTA Solution (10 mM Tris, pH 7.5, 0.1 mM EDTA) was added to the particles and mixed by pipetting 150 times while the sample was incubated for 5 minutes. The magnet was moved into position against the tube, and particles removed from the solution to the side of the tube. The solution containing DNA was aspirated and transferred to an output tube. The elution step was repeated a second time using 300 µL of Tris-EDTA Solution, and a third time using 100 µL Tris-EDTA Solution. The three elution volumes were combined into one final output tube.

*Results.* The purified DNA samples were analyzed by agarose gel electrophoresis (Gel 4). 18 µL of each sample was loaded onto a 1.4% agarose gel and electrophoresed at 100 V for 60 minutes. The gel shows clean, undegraded genomic DNA was purified from both sets of buffy coat samples.

The data in Graph 4 shows DNA yields in µg for the buffy coat samples with the higher and lower volumes of Lysis and Binding Solutions. The UV absorbance at 260 nm was used to calculate the DNA yields from each group. The results indicate that higher yields are obtained using the higher 2000 µL Lysis Solution and 3200 µL Binding Solution volumes. The results indicate that the automated methods provided consistent and competitive yields of clean and stable DNA from buffy coat, showing that the compact instrument of the present invention provides fast and effective methods for purifying DNA.

### Example 8 -Isolation and Purification of DNA from- Buffy Coat using the Direct Lysis Protocol

In this example, DNA from a 200 µL buffy coat sample was purified using the Direct Lysis protocol, but other volumes of buffy coat may also be purified using a similar protocol. This is an automated instrument protocol, but one or more of these steps can also be executed manually. In this example, 100 µL Promega Magnesil^{®} paramagnetic particles was added to a 10 mL conical Magnetic Processing Tube. Alternatively, 150 µL Micromod Sicaster-M-CT particles or 100 µL Novagen MagPrep particles could be used. A magnet was applied and the residual liquid from the particle solution was removed.

### Sample Lysis and Binding Steps

A volume of 4.5 µL Proteinase K Solution (20 mg/mL) was added to the Magnetic Processing Tube. A 200 µL buffy coat sample was added to the particle and Proteinase K mixture and loaded onto the instrument. 700 µL of Lysis Solution (2 M LiCl, 0.1% SDS, 30% DGME, 10 mM citrate) was added to the particles and mixed by pipetting 20 times. The sample was incubated at 45°C for 10 minutes with periodic mixing during the incubation. 800 µL of DNA Binding Solution (50 mM Tris, 10 M LiCl, 10% DGME) was added to the lysate and mixed by pipetting 5 times. The tube was incubated at room temperature for 10 minutes. The magnet was moved into position against the tube, and particles removed from the solution to the side of the tube. Waste was aspirated away from the particles into the waste tube and the magnet was removed from the tube.

*First Wash.* 1000 µL of Wash I Solution (55% ethanol, 5 M LiCl) was added to the particles and mixed by pipetting 15 times. The magnet was moved into position against the tube, and particles removed from the solution to the side of the tube. Waste was aspirated away from the particles into the waste tube and the magnet was removed from the tube. This wash step was repeated five times.

*Second Wash.* 1000 µL of 70% ethanol was added to the particles and mixed by pipetting 10 times. The magnet was moved into position against the tube, and particles removed from the solution to the side of the tube. Waste was aspirated away from the particles into the waste tube and the magnet was removed from the tube. This wash step was repeated five times.

*Final Wash.* 500 µL of Wash III Solution (70% ethanol, 100 mM Tris, 5 mM EDTA, pH 7) was added to the particles and mixed by pipetting 10 times. The magnet was moved into position against the tube, and particles removed from the solution to the side of the tube. Waste was aspirated away from the particles into the waste tube and the magnet was removed from the tube. This wash step was repeated five times.

*Sample Elution.* 150 - 200 µL of Tris-EDTA Solution (10 mM Tris, pH 7.5, 0.1 mM EDTA) was added to the particles and mixed by pipetting 5 times while the sample was incubated for 5 minutes. The magnet was moved into position against the tube, and particles removed from the solution to the side of the tube. The solution containing DNA was aspirated and transferred to an output tube. The elution step was repeated a second time using 50 - 200 µL of elution solution.

## Claims

1. An automated method for isolating DNA from a biological sample containing DNA, comprising the steps of:
(a) mixing a first reagent having a pH of at least 9, a sample, and magnetic particles in a magnetic processing tube such that the sample is lysed to release the nucleic acid in the sample, wherein the first reagent comprises (1) a lithium salt at a concentration of about 2-5 M; (2) a surfactant at a concentration of about 20-40% by volume; (3) a buffer; (4) a chelating agent at about 5-20 mM; (5) a detergent at about 0.05 - 2%; (6) an antifoaming agent at about 0.005-0.1% by volume, to form a lysate;
(b) optionally adding a second reagent to the lysate, such that the DNA quantitatively binds to the magnetic particles, wherein the second reagent comprises (1) a lithium salt at a concentration of at least 9 M (2) a surfactant at a concentration of at least 5%, and (3) a buffer; and
(c) applying a magnetic force to the magnetic processing tube; wherein the DNA is liberated from the sample.

2. An automated method for isolating DNA or RNA from a biological sample containing DNA or RNA, comprising the steps of:
(a) mixing a first reagent having a pH of at least 7, a sample, and magnetic particles in a magnetic processing tube such that the sample is lysed to release the nucleic acid in the sample to form a lysate, wherein the first reagent comprises (1) a lithium salt at a concentration of about 2-10 M; (2) a surfactant at a concentration of about 5-20% by volume; (3) a buffer; (4) a chelating agent at about 5-20 mM; (5) a detergent at about 0.05 - 5%; and (6) an antifoaming agent at about 0.005-0.1 % by volume;
(b) adding a second reagent having a pH of at least 7 to the lysate, such that the DNA binds to the magnetic particles, wherein the second reagent comprises either (1) a buffered binding solution comprising a lithium salt at a concentration of at least 5 M, a surfactant at a concentration of at least 5%, a buffer, and optionally an alcohol, or (2) an alcohol binding solution comprising lithium salt at a concentration of at least 5 M and an alcohol at a concentration of at least 35% v/v; and
(c) applying a magnetic force to the magnetic processing tube; wherein the DNA is liberated from the sample.

3. The method of claim 2, wherein the buffered binding solution comprises a lithium salt at a concentration of at least 9 M, a surfactant at a concentration of at least 10%, and a pH of 8.

4. The method of claim 2 or 3, wherein the alcohol binding solution comprises lithium salt at a concentration of at least 5 M and an alcohol at a concentration of at least 55% v/v.

5. A lysis solution for lysing cells comprising a alkali metal salt at a concentration of about 2 M to about 10 M, a detergent at a concentration of 0.05 to 2.0% w/v, a surfactant at a concentration of about 5 to 40% w/v, a chelating agent at a concentration of about 5 to 20 mM, a buffer, and an antifoam agent of about 0.005 % to about 0.1 %, wherein the solution has a pH of at least 7.

6. The solution of claim 5, wherein the solution has a pH of at least 9, preferably of at least 11.

7. The solution of claim 5 or 6, wherein the alkali metal salt is lithium chloride or lithium bromide.

8. The solution of any of claims 5 to 7, wherein the detergent is a non-ionic detergent, preferably a Tween class detergent, a Triton class detergent, a Tergitol detergent, Nonidets or lgepal, or an anionic detergent, preferably SDS (sodium dodecyl sulfate).

9. The solution of any of claims 5 to 8, wherein the surfactant is diethylene glycol monoethyl ether (DGME).

10. The solution of any of claims 5 to 9, wherein the antifoaming agent is Dow Corning^{®} Antifoam A compound.

11. The solution of any of claims 5 to 10, wherein the buffer is Tris buffer.

12. The solution of any of claims 5 to 11, wherein the lithium salt is present at a concentration of about 2 M to about 5 M.

13. The solution of claim 5, comprising 50 mM Tris (pH 10-11), 2 M LiCl, 0.1% SDS, 30% DGME, 10 mM citrate and 0.05% Dow Corning^{®} Antifoam A compound.

14. The solution of claim 5, comprising 100 mM Tris (pH 7), 8 M LiCl, 0.1% Triton X, 10% DGME, 10 mM EDTA and 0.01% Dow Corning^{®} Antifoam A compound.

15. The solution of claim 14, further comprising 20 mM TCEP.

## Patentansprüche

1. Ein automatisiertes Verfahren zum Isolieren von DNA aus einer DNA-haltigen biologischen Probe, umfassend die Schritte:
(a) Mischen eines ersten Reagenzes mit einem pH von wenigstens 9, einer Probe und magnetischen Partikeln in einem eine magnetische Verarbeitung zulassenden Röhrchen, so dass die Probe lysiert wird, um die Nucleinsäure in der Probe freizusetzen, wobei das erste Reagenz (1) ein Lithiumsalz in einer Konzentration von ungefähr 2-5 M; (2) ein oberflächenaktives Mittel in einer Konzentration von ungefähr 20-40 Vol.-%; (3) einen Puffer; (4) einen Chelatbildner zu ungefähr 5-20 mM; (5) ein Detergens zu ungefähr 0,05 - 2%; (6) ein Antischaummittel zu ungefähr 0,005-0,1 Vol.-% enthält, um ein Lysat zu bilden;
(b) optional das Zugeben eines zweiten Reagenzes zu dem Lysat, so dass die DNA quantitativ an die magnetischen Partikel bindet, wobei das zweite Reagenz (1) ein Lithiumsalz in einer Konzentration von ungefähr 9 M, (2) ein oberflächenaktives Mittel in einer Konzentration von wenigstens 5% und (3) einen Puffer enthält; und
(c) Anlegen einer magnetischen Kraft an das eine magnetische Verarbeitung zulassende Röhrchen, wobei die DNA aus der Probe freigesetzt wird.

2. Ein automatisiertes Verfahren zum Isolieren von DNA oder RNA aus einer DNA-haltigen biologischen Probe, umfassend die Schritte:
(a) Mischen eines ersten Reagenzes mit einem pH von wenigstens 7, einer Probe und magnetischen Partikeln in einem eine magnetische Verarbeitung zulassenden Röhrchen, so dass die Probe lysiert wird, um die Nucleinsäure in der Probe freizusetzen, um ein Lysat zu bilden, wobei das erste Reagenz (1) ein Lithiumsalz in einer Konzentration von ungefähr 2-10 M; (2) ein oberflächenaktives Mittel in einer Konzentration von ungefähr 5-20 Vol.-%; (3) einen Puffer; (4) einen Chelatbildner zu ungefähr 5-20 mM; (5) ein Detergens zu ungefähr 0,05 - 5% und (6) ein Antischaummittel zu ungefähr 0,005-0,1 Vol.-% enthält;
(b) Zugeben eines zweiten Reagenzes mit einem pH von wenigstens 7 zu dem Lysat, so dass die DNA an die magnetischen Partikel bindet, wobei das zweite Reagenz entweder (1) eine gepufferte Bindelösung, die ein Lithiumsalz in einer Konzentration von wenigstens 5 M, ein oberflächenaktives Mittel in einer Konzentration von wenigstens 5%, einen Puffer und optional einen Alkohol enthält, oder (2) eine Alkoholbindelösung, die ein Lithiumsalz in einer Konzentration von wenigstens 5 M und einen Alkohol in einer Konzentration von wenigstens 35% v/v enthält, enthält; und
(c) Anlegen einer magnetischen Kraft an das eine magnetische Verarbeitung zulassende Röhrchen, wobei die DNA aus der Probe freigesetzt wird.

3. Das Verfahren gemäß Anspruch 2, wobei die gepufferte Bindelösung ein Lithiumsalz in einer Konzentration von wenigstens 9 M, ein oberflächenaktives Mittel mit einer Konzentration zu wenigstens 10% enthält und einen pH von 8 aufweist.

4. Das Verfahren gemäß Anspruch 2 oder 3, wobei die Alkoholbindelösung ein Lithiumsalz in einer Konzentration von wenigstens 5 M und einen Alkohol in einer Konzentration von wenigstens 55% v/v enthält.

5. Eine Lyselösung zum Lysieren von Zellen, enthaltend ein Alkalimetallsalz in einer Konzentration von ungefähr 2 M bis ungefähr 10 M, ein Detergens in einer Konzentration von 0,05 - 2,0% w/v, ein oberflächenaktives Mittel in einer Konzentration von ungefähr 5-40% w/v, einen Chelatbildner in einer Konzentration von ungefähr 5-20 mM, einen Puffer und ein Antischaummittel zu ungefähr 0,005% bis ungefähr 0,1%, wobei die Lösung einen pH von wenigstens 7 hat.

6. Die Lösung gemäß Anspruch 5, wobei die Lösung einen a pH von wenigstens 9, bevorzugt von wenigstens 11 hat.

7. Die Lösung gemäß Anspruch 5 oder 6, wobei das Alkalimetallsalz Lithiumchlorid oder Lithiumbromid ist.

8. Die Lösung gemäß einem der Ansprüche 5 bis 7, wobei das Detergens ein nichtionisches Detergens, bevorzugt ein Detergens der Tween-Klasse, ein Detergens der Triton-Klasse, ein Tergitol-Detergens, Nonidets oder Igepal oder ein anionisches Detergens, bevorzugt SDS (Natriumdodecylsulfat) ist.

9. Die Lösung gemäß einem der Ansprüche 5 bis 8, wobei das oberflächenaktive Mittel Diethylenglykolmonoethylether (DGME) ist.

10. Die Lösung gemäß einem der Ansprüche 5 bis 9, wobei das Antischaummittel die Verbindung Dow Corning^{®} Antifoam A ist.

11. Die Lösung gemäß einem der Ansprüche 5 bis 10, wobei der Puffer Tris-Puffer ist.

12. Die Lösung gemäß einem der Ansprüche 5 bis 11, wobei das Lithiumsalz in einer Konzentration von ungefähr 2 M bis ungefähr 5 M vorliegt.

13. Die Lösung gemäß Anspruch 5, enthaltend 50 mM Tris (pH 10-11), 2 M LiCl, 0,1% SDS, 30% DGME, 10 mM Citrat und 0,05% Dow Corning^{®} Antifoam A Verbindung.

14. Die Lösung gemäß Anspruch 5, enthaltend 100 mM Tris (pH 7), 8 M LiCl, 0,1% Triton X, 10% DGME, 10 mM EDTA und 0,01% Dow Corning^{®} Antifoam A Verbindung.

15. Die Lösung gemäß Anspruch 14, außerdem enthaltend 20 mM TCEP.

## Revendications

1. Procédé automatisé pour isoler de l'ADN d'un échantillon biologique contenant de l'ADN, comprenant les étapes consistant à :
(a) mélanger un premier réactif ayant un pH d'au moins 9, un échantillon et des particules magnétiques dans un tube de traitement magnétique de sorte que l'échantillon soit lysé pour libérer l'acide nucléique dans l'échantillon, dans lequel le premier réactif comprend (1) un sel de lithium à une concentration d'environ 2-5 M ; (2) un tensioactif à une concentration d'environ 20-40 % en volume ; (3) un tampon ; (4) un agent chélatant à environ 5-20 mM ; (5) un détergent à environ 0,05-2 % ; (6) un agent anti-mousse à environ 0,005-0,1 % en volume, pour former un lysat ;
(b) ajouter éventuellement un second réactif au lysat, de sorte que l'ADN se lie quantitativement aux particules magnétiques, dans lequel le second réactif comprend (1) un sel de lithium à une concentration d'au moins 9 M, (2) un tensioactif à une concentration d'au moins 5 %, et (3) un tampon ; et
(c) appliquer une force magnétique au tube de traitement magnétique ; dans lequel l'ADN est libéré de l'échantillon.

2. Procédé automatisé d'isolation de l'ADN ou de l'ARN d'un échantillon biologique contenant de l'ADN ou de l'ARN, comprenant les étapes consistant à :
(a) mélanger un premier réactif ayant un pH d'au moins 7, un échantillon et des particules magnétiques dans un tube de traitement magnétique de sorte que l'échantillon soit lysé pour libérer l'acide nucléique dans l'échantillon, afin de former un lysat, dans lequel le premier réactif comprend (1) un sel de lithium à une concentration d'environ 2-10 M ; (2) un tensioactif à une concentration d'environ 5-20 % en volume ; (3) un tampon ; (4) un agent chélatant à environ 5-20 mM ; (5) un détergent à environ 0,05-5 % ; et (6) un agent anti-mousse à environ 0,005-0,1 % en volume ;
(b) ajouter un second réactif, ayant un pH d'au moins 7 au lysat, de sorte que l'ADN se lie aux particules magnétiques, dans lequel le second réactif comprend (1) une solution de liaison tampon comprenant un sel de lithium à une concentration d'au moins 5 M, un tensioactif à une concentration d'au moins 5 %, un tampon et éventuellement un alcool, ou (2) une solution de liaison d'alcool comprenant un sel de lithium à une concentration d'au moins 5 M et un alcool à une concentration d'au moins 35 % v/v ; et
(c) appliquer une force magnétique au tube de traitement magnétique ; dans lequel l'ADN est libéré de l'échantillon.

3. Procédé selon la revendication 2, dans lequel la solution de liaison tamponnée comprend un sel de lithium à une concentration d'au moins 9 M, un tensioactif à une concentration d'au moins 10 %, et un pH de 8.

4. Procédé selon la revendication 2 ou 3, dans lequel la solution de liaison d'alcool comprend un sel de lithium à une concentration d'au moins 5 M et un alcool à une concentration d'au moins 55 % v/v.

5. Solution de lyse pour lyser les cellules comprenant un sel de métal alcalin à une concentration d'environ 2 M à environ 10 M, un détergent à une concentration de 0,05 à 2,0 % m/v, un tensioactif à une concentration d'environ 5 à 40 % m/v, un agent chélatant à une concentration d'environ 5 à 20 mM, un tampon et un agent anti-mousse d'environ 0,005 % à environ 0,1 %, dans laquelle la solution a un pH d'au moins 7.

6. Solution selon la revendication 5, dans laquelle la solution a un pH d'au moins 9, de préférence d'au moins 11.

7. Solution selon la revendication 5 ou 6, dans laquelle le sel de métal alcalin est un chlorure de lithium ou un bromure de lithium.

8. Solution selon l'une quelconque des revendications 5 à 7, dans laquelle le détergent est un détergent non-ionique, de préférence un détergent de classe Tween, un détergent de classe Triton, un détergent Tergitol, Nonidets ou Igepal, ou un détergent anionique, de préférence du SDS (sodium dodécyl sulfate).

9. Solution selon l'une quelconque des revendications 5 à 8, dans laquelle le tensioactif est le monoéthyléther de diéthylène glycol (DGME).

10. Solution selon l'une quelconque des revendications 5 à 9, dans laquelle l'agent anti-mousse est un composé anti-mousse A de Dow Corning®.

11. Solution selon l'une quelconque des revendications 5 à 10, dans laquelle le tampon est un tampon Tris.

12. Solution selon l'une quelconque des revendications 5 à 11, dans laquelle le sel de lithium est présent à une concentration d'environ 2 M à environ 5 M.

13. Solution selon la revendication 5, comprenant 50 mM de Tris (pH 10-11), 2 M de LiCl, 0,1 % de SDS, 30 % de DGME, 10 mM de citrate et 0,05 % de composé Anti-mousse A de Dow Corning®.

14. Solution selon la revendication 5, comprenant 100 mM de Tris (pH 7), 8 M de LiCl, 0,1 % de Triton X, 10 % de DGME, 10 mM d'EDTA et 0,01 % de composé Anti-Mousse A Dow Corning®.

15. Solution selon la revendication 14, comprenant en outre 20 mM de TCEP.
